# EUROPÄISCHE PATENTSCHRIFT

(11) **EP 2 168 957 B1**
(45) Veröffentlichungstag und Bekanntmachung des Hinweises auf die Patenterteilung: **15.02.2012**
(21) Anmeldenummer: 09176696.4
(22) Anmeldetag: 20.11.2009
(51) Int. Cl.: C07D 309/04, A23L 2/39, A61K 8/49, A61K 31/351, A61Q 11/00, A23G 4/06, A23G 3/36, A61P 43/00

(54) **Alkylsubstituierte Tetrahydropyrane als Aromastoffe**
Alkyl-substituted tetrahydropyranes as aromatic materials
Tétrahydropyrane substitué par alkyl en tant qu'arome

(30) Priorität: 06.03.2009 US 158162 P
(43) Veröffentlichungstag der Anmeldung: 31.03.2010
(73) Patentinhaber: Symrise AG, 37603 Holzminden (DE)
(72) Erfinder: Oertling, Heiko, 37603, Holzminden (DE); Brocke, Constanze, 64521, Groß-Gerau (DE); Loges, Hubert, 37671, Höxter (DE); Machinek, Arnold, 37603, Holzminden (DE)
(74) Vertreter: Eisenführ, Speiser & Partner

(56) Entgegenhaltungen:
- EP-A1- 1 847 181
- DATABASE CAPLUS [Online] CHEMICAL ABSTRACTS SERVICE, COLUMBUS, OHIO, US; 1967, VOITSEKHOVSKAYA, A. L. ET AL: "Substituted lactones and their transformations. VII. Synthesis of 2-methyl-3-alkyltetrahydropyrans and 3-alkyl-2,6-hexanediols by reduction of .delta.-methyl-.gamma.-alkyl-.delta.-valer olactones" XP002582475 gefunden im STN Database accession no. 1967:55320
- KULA JOZEF ET AL: "Tetrahydrofuran and tetrahydropyran derivatives as odor substances" PERFUMER AND FLAVORIST, Bd. 17, Nr. 5, 1992, Seiten 77-79, 82, 84, XP009133547 & 12TH INTERNATIONAL CONGRESS OF FLAVORS, FRAGRANCES, AND ESSENTIAL OILS, VIENNA, AUSTRIA, OCTOBER 4-8 ISSN: 0272-2666

## Beschreibung

Die vorliegende Erfindung betrifft alkylsubstituierte Tetrahydropyrane der nachfolgenden Formel (I), Mischungen umfassend diese alkylsubstituierten Tetrahydropyrane, deren jeweilige Verwendung und entsprechende aromatisierte Produkte.

In einer besonderen Ausführungsform betrifft die Erfindung die Verwendung dieser Aromastoffe in Aroma- und Geschmacksstoffkompositionen mit Frischewirkung für den Einsatz in Mundhygieneprodukten.

Die Erfindung betrifft desweiteren Zubereitungen und Rhinologica, die im Bereich des Mundes, des Rachens und der Atemwege ein frisches und befreiendes Gefühl hervorrufen und Zubereitungen, die diese Verbindungen enthalten.

Um dem Bedarf des Verbrauchers nach immer neuen Geruchs- und Geschmackserlebnissen nachzukommen, besteht in der Aromen- und Geschmacksstoffindustrie ein großer Bedarf an Stoffen, die herausragende sensorische (d.h. mit den Sinnen wahrnehmbare) Eigenschaften besitzen und mit denen sich bemerkenswerte neuartige Effekte erzielen lassen. Dabei können neben den reinen geruchlichen und geschmacklichen Eigenschaften weitere zusätzliche Eigenschaften von Bedeutung sein, so z.B. dass die Geruchs- und Geschmacksempfindungen gehemmt oder verstärkt werden.

Aroma-, bzw. Geschmacksstoffkompositionen mit Frischewirkung verleihen z.B. Mundhygieneprodukten, wie Zahnpasten und Mundwässern, und Süßwaren wie Bonbons und Kaugummis, ihren typischen, frischen und als angenehm empfundenen Geschmack.

Stoffe, die in großem Umfang für die Herstellung solcher Aroma-, bzw. Geschmacksstoffkompositionen mit Frischewirkung verwendet werden, sind beispielsweise Eucalyptol (1,8-Cineol) und Menthol. Die Verwendung dieser Stoffe ist jedoch mit einigen Nachteilen behaftet. So zeigt das Eucalyptol neben seiner Frischewirkung einen sehr starken medizinischen Eigengeschmack, der von vielen Konsumenten als abstoßend empfunden wird, besonders wenn das Eucalyptol in höheren Dosierungen verwendet wird. Bei der Verwendung von Menthol setzt die Frischewirkung mit einer gewissen Verzögerung ein, und bei höheren Dosierungen entwickelt das Menthol einen bitter-scharfen Eigengeschmack, der eine eher unangenehme Wirkung hat.

Es war daher die primäre Aufgabe der vorliegenden Erfindung, Verbindungen bzw. Gemische von Verbindungen anzugeben, die in Aroma- und/oder Geschmacksstoffkompositionen mit Frischewirkung zu einem verstärkten, schnell wahrnehmbaren Frischeerlebnis führen. Darüber hinaus sollten die anzugebenden Verbindungen bzw. Verbindungsgemische vorzugsweise einen möglichst schwachen Eigengeschmack zeigen, insbesondere wenig oder gar nicht medizinisch und/oder bitter schmecken.

Die erfindungsgemäßen neuen Tetrahydropyrane zeigen eine dem 1,8-Cineol (Eucalyptol) verwandte Wirkung in Bezug auf ein frisches befreiendes Gefühl im Mund, Rachenraum und den Atemwegen zu erzielen, ohne dabei ein unangenehmes Geschmacksempfinden zu erzeugen.

Die erfindungsgemäßen Tetrahydropyrane zeichnen sich im wesentlichen durch einen neutralen, frisch-kühlen Geschmack aus. Sie eignen sich deshalb hervorragend dazu, in Aroma- und Geschmacksstoffkompositionen eingesetzt zu werden. Bemerkenswert ist ihre Fähigkeit, in Aroma- und Geschmacksstoffkompositionen die Intensität und die Kraft des Frische-Gefühls zu steigern.

Die Wirkung, ein frisches befreiendes Gefühl im Mund, Rachenraum und den Atemwegen hervorzurufen, trifft bei den erfindungsgemäßen alkylierten Tetrahydropyranen auf alle isomeren Formen, d. h. Diastereomere und Enantiomere zu.

Erfindungsgemäß wird deshalb ein alkyliertes Tetrahydropyran der Formel (I) angegeben: 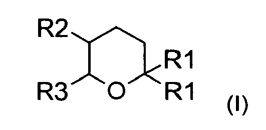 wobei
jedes R1 unabhängig voneinander entweder
Wasserstoff
oder ein verzweigter oder unverzweigter Alkylrest mit 1 bis 3 C-Atomen
oder ein verzweigter oder unverzweigter Alkenylrest mit 2 bis 3 C-Atomen,
und R2 ein verzweigter oder unverzweigter Alkyl oder Alkenylrest mit 3 bis 4 C-Atomen
und R3 ein verzweigter oder unverzweigter Alkylrest mit 1 bis 5 C-Atomen ist,
ausgenommen
2,6-Dimethyl-3-(1-methylethenyl)-tetrahydropyran (CA5-Nr 1008981-43-8): 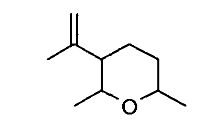 6-Methyl-3-(1-methylethenyly2-(2-methylpropylytetrahydropyran (CAS-Nr 1005159-81-8): 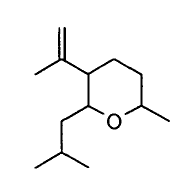 2-Methyl-3-n-propyl-tetrahydropyran (CAS-Nr 13687-01-9): 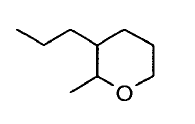 und 2-Methyl-3-n-butyl-tetrahydropyran (CAS-Nr 13687-02-0):

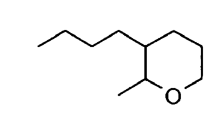

Alkylierte Tetrahydropyranderivate sind als sensorisch aktive Substanzen unter anderem beispielsweise in Perfumer & Flavorist, Vol 17, 1992, 77-92 und in PAFAI Journal (1984), 6(3), 15-18 beschrieben.

Als Aromastoffe finden aus dieser Strukturklasse vor allen Dingen Rosenoxid [2-(2-Methyl-1-propenyl)-4-methyltetrahydropyran, CAS-Nr 16409-43-1; FEMA-Nr 3236] und Limetol (2-Vinyl-2,6,6-trimethyltetrahydropyran, CAS-Nr 7392-19-0; FEMA-Nr 3735) Verwendung; für Limetol beispielsweise beschrieben in US 5,137,741. Für beide Substanzen ist eine geschmacksverstärkende Wirkung in Aromen belegt: Für Limetol ist in US 5,589,158 eine geschmacksverstärkende Wirkung für Mintaromen in Kombination mit Nerylacetat und Benzylbenzoat beschrieben; für Rosenoxid ist eine geschmacksmodulierende Wirkung in vergorenen Getränken in DE 698 24 198 T2 beschrieben. Sowohl Rosenoxid, als auch Limetol besitzen aber ein auffälliges charakteristisches Geschmacksprofil, welches ihren Einsatz für gewisse Anwendungbereiche als geschmacksmodulierende Substanzen einschränkt oder sogar ganz verhindert.

Für den Mundhygienebereich sind geschmacksmodulierende Substanzen bekannt; so werden in DE 102 25 350 A1 die alkylierten 1,3-Dioxane als Aromastoffe beschrieben, die frischeverstärkende und aromaintensivierende Eigenschaften besitzen. Ein Nachteil dieser Verbindungklasse ist allerdings die begrenzte Stabilität in verschiedenen Medien, wie beispielsweise Zahnpasta.

Ebenfalls aromaintensivierende Eigenschaften besitzen die in WO 02/41861 A1 beschriebenen acyclischen Ether. Im Gegensatz zu den hier beschriebenen cyclischen Ethern setzt deren Wirkung allerdings nicht augenblicklich ein, sondern mit einer gewissen zeitlichen Verzögerung. Der Vorteil aller erfindungsgemäßen cyclischen Ether liegt dagegen in der Unmittelbarkeit ihrer Wirkung ("Boost-Effekt").

Innerhalb der Gruppe erfindungsgemäßer Verbindungen und von Mischungen bestehend aus oder enthaltend zwei, drei, vier oder mehr der Verbindungen sind bestimmte alkylierte Tetrahydropyrane bevorzugt. Bevorzugt ist es insbesondere, wenn die Verbindung bzw. eine der Verbindungen ausgewählt ist aus solchen alkylierten Tetrahydropyranen der Formel (I), bei denen
jedes R1 unabhängig voneinander ein Methyl-, Ethyl- oder Vinylrest ist,
R2 ein Isopropyl-, Isopropenyl-, sec-Butyl- oder sec-Butenylrest ist, und
R3 ein unverzweigter Alkylrest mit 1 bis 3 C-Atomen ist.

Hierbei ist die Verbindung bzw. zumindest eine der Verbindungen der Mischung bevorzugt ein solches alkyliertes Tetrahydropyran, bei dem
jedes R1 unabhängig voneinander ein Methyl- oder Ethylrest ist,
R2 ein Isopropyl- oder sec-Butylrest ist, und
R3 ein Methyl- oder Ethylrest ist.

Vorzugsweise ist die Verbindung bzw. eine der Verbindungen der Mischung ausgewählt aus der Gruppe bestehend aus 2,6-Diethyl-5-isopropyl-2-methyltetrahydropyran, 6-Ethyl-5-isopropenyl-2-methyl-2-vinyltetrahydropyran, 2,6-Dimethyl-5-isopropyl-2-ethyltetrahydropyran, 2,6-Dimethyl-5-isopropenyl-2-vinyltetrahydropyran, 2,6-Diethyl-5-sec.-butyl-2-methyltetrahydropyran und 2,6-Dimethyl-5-sec.-butyl-2-ethyltetrahydropyran.

Eine ganz besonders bevorzugte Verbindung zum Lösen der Aufgabe der Erfindung ist 2,6-Diethyl-5-isopropyl-2-methyltetrahydropyran; entsprechend bevorzugt sind auch erfindungsgemäße Mischungen enthaltend zumindest diese Verbindung.

Die Erfindung beruht auf der überraschenden Erkenntnis, dass erfindungsgemäße alkylierte Tetrahydropyrane die Frischewirkung von Aromakompositionen (Kompositionen enthaltend einen oder mehrere Aromastoffe und/oder Geschmacksstoffe) mit Frischewirkung schnell wahrnehmbar verstärken, ohne jedoch einen starken Eigengeschmack aufzuweisen.

Die erfindungsgemäßen Mischungen bestehen aus zwei, drei, vier oder mehr Verbindungen der Formel (I), vorzugsweise jeweils in einer der vorstehend als bevorzugt angegebenen Ausgestaltungen. Des Weiteren bevorzugt ist es, wenn in einer erfindungsgemäß zu verwendenden Mischung nicht nur eine vorstehend als besonders bevorzugt bezeichnete Verbindung der Formel (I) vorhanden ist, d. h. nicht nur eine Verbindung, die eine, mehrere oder ausschließlich besonders bevorzugte Gruppen R1, R2 und R3 umfasst, sondern zwei oder mehr als besonders bevorzugte erfindungsgemäße Verbindungen der Formel (I). Vorzugsweise sind somit zwei, drei oder sämtliche Verbindungen in einer erfindungsgemäßen Mischung bestehend aus Verbindungen der Formel (I) aus der Gruppe der als bevorzugt bezeichneten Verbindungen ausgewählt.

Die Verbindungen der Formel (I) können gemäß den dem Fachmann bekannten Herstellungsmethoden erhalten werden. Beispielsweise können die Verbindungen der Formel (I) in einem Verfahren hergestellt werden, wie in dem nachfolgenden Schema verdeutlicht ist. Die Umsetzung des Aldehyds (B) mit der Alkoholkomponente (A) führt zu dem entsprechenden cyclisierten Tetrahydropyranverbindung (C), welches unter Formel (I) zu subsumieren ist. Tetrahydropyranverbindung (C) kann optional in einem zweiten Reaktionsschritt zu der Tetrahydropyranverbindung (D) reduziert werden, welche ebenfalls unter Formel (I) zu subsumieren ist. 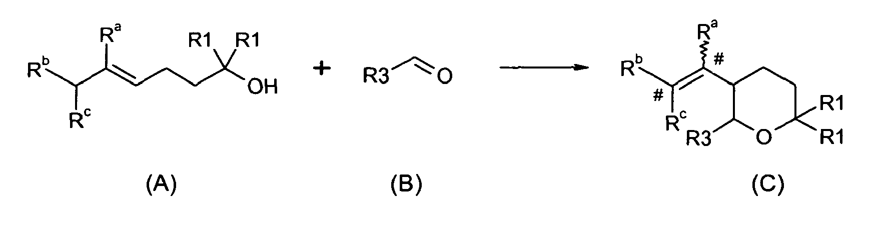 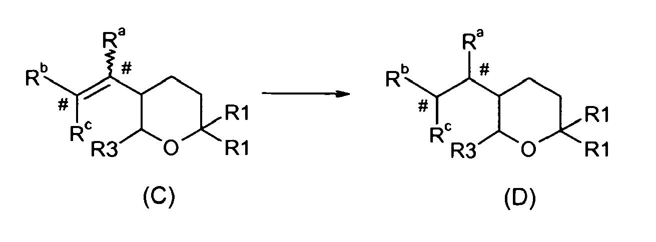 wobei
R1 und R3 jeweils die oben genannte (gegebenenfalls bevorzugte) Bedeutung haben, und
R^{a}, R^{b} und R^{c} unabhängig voneinander Wasserstoff, Methyl oder Ethyl darstellen,
mit der Maßgabe, dass R^{a}, R^{b} und R^{c} in Summe 1 oder 2 C-Atome enthalten, d.h. dass die Reste R^{a}, R^{b} und R^{c} zusammen mit den beiden mit #-markierten CH-Resten insgesamt dem Rest R2 in Formel (I) entsprechen.

Die Umsetzung von Alkohol (A) mit Aldehyd (B) erfolgt vorzugsweise in Gegenwart saurer Katalysatoren. Als saure Katalysatoren können z.B. anorganische Säuren wie Phosphorsäure, Polyphosphorsäure, Salzsäure, Salpetersäure, Schwefelsäure, organische Säuren wie Methansulfonsäure, Toluolsulfonsäure oder Trifluoressigsäure eingesetzt werden. Daneben können auch feste Säuren wie saure Ionentauscher, saure Aluminosilicate oder saure Zeolithe verwendet werden.

Weitere bevorzugte saure Katalysatoren sind Lewis-Säuren wie beispielsweise AlX₃, ZnX₂, FeX₃, TiX₄, wobei X für ein Halogenid aus der Gruppe Chlor, Brom oder Iod steht, sowie BF₃, insbesondere AlCl₃, ZnCl₂, FeCl₃, TiCl₄ sowie BF₃-Addukte.

Besonders bevorzugte Katalysatoren sind Schwefelsäure, Phosphorsäure und BF₃-Addukte, insbesondere BF₃-Etherat.

Das molare Verhältnis von Alkohol (A) zu Aldehyd (B) liegt bevorzugt im Bereich von 1 : 5 bis 1 : 3, besonders bevorzugt im Bereich von 1 : 3 bis 1 : 1. Insbesondere bevorzugt ist ein molares Verhältnis von Alkohol (A) zu Aldehyd (B) von 1 zu 1,05 - 1,2 (d.h. leichter Überschuss an Aldehyd).

Die Reaktionstemperatur liegt vorzugsweise im Bereich von -20°C bis 50°C, bevorzugt im Bereich von -10°C bis 40°C und insbesonders bevorzugt im Bereich von 0°C bis 15°C.

Besonders bevorzugt ist bei der Umsetzung von (A) mit (B) folgendes Temperaturprofil: zunächst erfolgt die Reaktion der Alkoholkomponente (A) mit der Aldehydkomponente (B) in der Kälte (vorzugsweise bei 0 - 10°C). Im Falle der Katalyse mit einer Protonensäure folgt vorzugsweise anschließend ein Erwärmen der Reaktionsmischung auf etwa 60 - 80°C, um eine möglichst vollständige Umsetzung zu erreichen. Ein solches anschließendes Erwärmen kann bei Katalyse mit einer Lewissäure hingegen entfallen.

Verbindungen der Formel (D), dabei insbesondere solche Verbindungen der Formel (I), die keine C-C-Doppelbindungen aufweisen, können mittels Reduktion aus den entsprechenden ungesättigten Verbindungen der Formel (C) erhalten werden, vorzugsweise mittels Hydrierung an Hydrierkatalysatoren in einer Wasserstoffatmosphäre.

Als Hydrierkatalysatoren zur Reduktion der in Verbindungen der Formel (C) vorhandenen C-C-Doppelbindung(en) eignen sich beispielsweise Elemente der 8. Nebengruppe des Periodensystems. Besonders vorteilhaft sind hier die Elemente Nickel, Palladium, Platin, Rhodium, Iridium, Ruthenium sowie deren Mischungen, Verbindungen und Legierungen. Diese Katalysatoren können, vorzugsweise in elementarer und fein verteilter Form, aufgebracht auf Träger oder zusammen mit anderen Metallen oder deren Verbindungen, eingesetzt werden. Als vorteilhafte Trägermaterialen seien genannt Aktivkohle, Aluminiumoxide, Metalloxide, Kieselgele, Zeolithe, Tone, Tongranulate, amorphe Aluminiumsilicate, oder sonstige anorganische oder polymere Träger.

Der Wasserstoffdruck bei der Hydrierungsreaktion liegt im Bereich von 1 bis 200 bar, bevorzugt im Bereich von 1 bis 100 bar, insbesondere bevorzugt im Bereich von 5 bis 50 bar.

Verbindungen der Formel (I) können nach üblichen Methoden, z.B. durch Destillation, gereinigt werden.

Zur Erzielung von frischen, etherischen, minzigen, kühlenden, süßlichen und fruchtigen Geschmacksnoten in Verbindung mit einem frischen befreienden Gefühl im Mund, Rachenraum und den Atemwegen können die erfindungsgemäßen Tetrahydropyrane in reiner Form, miteinander oder aber in einer besonders bevorzugten Form mit Aroma- und/oder Geschmacksstoffen kombiniert werden.

Bevorzugt sind deshalb auch Mischungen umfassend oder bestehend aus
a) einem, zwei, drei, vier oder mehr erfindungsgemäßen alkylierten Tetrahydropyranen,
b) einem oder mehreren (flüchtigen) Aroma- und/oder Geschmacksstoffen, insbesondere einer oder mehreren Substanz(en) ausgewählt aus der Gruppe bestehend aus Substanzen mit physiologischer Kühlwirkung, Aromastoffe ohne physiologische Kühlwirkung, trigeminal oder mundwässernd wirksame Substanzen ohne physiologische Kühlwirkung, und geschmacksmodulierende Substanzen,
c) und optional einem kosmetisch oder pharmazeutisch akzeptablen Träger.

Der Begriff Aromastoff(e) wird in der vorliegenden Erfindung im engeren Sinne entsprechend der Richtlinie des Rates 88/388/EWG vom 22. Juni 1988, veröffentlicht im ABI. L 184 vom 15. Juli 1988, S. 61 verwendet. Aromastoffe sind nach dieser Richtlinie:

"Definierte chemische Stoffe mit Aromaeigenschaften, die wie folgt gewonnen werden:
i) durch geeignete physikalische Verfahren (einschließlich Destillation und Extraktion mit Lösungsmitteln) oder enzymatische bzw. mikrobiologische Verfahren aus Stoffen pflanzlichen oder tierischen Ursprungs, die als solche verwendet oder mittels herkömmlicher Lebensmittelzubereitungsverfahren (einschließlich Trocknen, Rösten und Fermentierung) für den menschlichen Verzehr verarbeitet werden;
ii) durch chemische Synthese oder durch Isolierung mit chemischen Verfahren, wobei ihre chemische Beschaffenheit mit einer Substanz identisch ist, die in einem Stoff pflanzlichen oder tierischen Ursprungs im Sinne von Ziffer i) natürlich vorkommt;
iii) durch chemische Synthese, wobei jedoch ihre chemische Beschaffenheit nicht mit einer Substanz identisch ist, die in einem Stoff pflanzlichen oder tierischen Ursprungs im Sinne von Ziffer i) natürlich vorkommt."

Beispiele erfindungsgemäß einzusetzender Aromastoffe sind aufgelistet in den Abschnitten 1 bis 3 der Entscheidung der Kommission vom 23. Februar 1999 über ein Verzeichnis der in oder auf Lebensmitteln verwendeten Aromastoffe, das gemäß Verordnung (EG) Nr. 2232/96 des Europäischen Parlaments und des Rates vom 28. Oktober 1996 erstellt wurde (1999/217/EG), einzusehen im Amtsblatt der Europäischen Gemeinschaften L 84/1 vom 27. März 1999 sowie im Anhang der Entscheidung der Kommission vom 18. Juli 2000 zur Änderung der Entscheidung 1999/217/EG der Kommission über ein Verzeichnis der in oder auf Lebensmitteln verwendeten Aromastoffe (2000/489/EG), einzusehen im Amtsblatt der Europäischen Gemeinschaften L 197/53 vom 3. August 2000.

Unter einem flüchtigen Aromastoff wird im Rahmen der vorliegenden Erfindung vorzugsweise eine sensorisch wirksame Komponente mit einem Dampfdruck von größer oder gleich 0,01 Pa bei 25°C, insbesondere mit einem Dampfdruck von größer oder gleich 0,025 Pa bei 25°C, verstanden. Ein Großteil der flüchtigen Aromastoffe weist einen Dampfdruck von größer oder gleich 1 Pa bei 25°C auf, so dass im Speziellen solche Stoffe als Aromastoffe im Sinne der vorliegenden Erfindung angesehen werden.

Nachfolgend wird insbesondere der Begriff "Kühlsubstanz" zur Bezeichnung von physiologisch wirksamen Kühlsubstanzen (Kühlwirkstoffen) verwendet. Kühlsubstanzen werden regelmäßig eingesetzt, um einen kühlen sensorischen Eindruck auf der Haut bzw. Schleimhaut, zum Beispiel auf der Schleimhaut im Mund-, Nasen- und/oder Rachenraum hervorzurufen, wobei allerdings tatsächlich keine physikalische Abkühlung wie zum Beispiel bei der Verdunstung von Lösungsmitteln stattfindet. Als Kühlsubstanzen können sowohl Einzelkomponenten als auch Gemische verwendet werden. Die bekannteste Kühlsubstanz ist L-Menthol.

Als weitere Aromastoffe - mit oder ohne physiologische Kühlwirkung - eignen sich sowohl komplexe natürliche Rohstoffe wie aus Pflanzen gewonnene Extrakte und etherische Öle, bzw. daraus gewonnene Fraktionen und einheitliche Stoffe, als auch einheitliche synthetisch oder biotechnologisch gewonnene Aromastoffe.

Beispiele für natürliche Rohstoffe sind Pfefferminzöle, Krauseminzöle, Mentha-Arvensis-Öle, Anisöle, Nelkenöle, Citrusöle, Zimtrindenöle, Wintergrünöle, Cassiaöle, Davanaöle, Fichtennadelöle, Eucalyptusöle, Fenchelöle, Galbanumöle, Ingweröle, Kamillenöle, Kümmelöle, Rosenöle, Geraniumöle, Salbeiöle, Petersilienöle, Scharfgarbenöle, Sternanisöle, Thymianöle, Wacholderbeeröle, Rosmarinöle, Angelikawurzelöle, und die Fraktionen dieser Öle.

Beispiele für einheitliche Aromastoffe sind Anethol, Menthol, Menthon, Isomenthon, Menthylacetat, Menthofuran, Mintlacton, Eucalyptol, Limonen, Eugenol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Isoamylacetat, Isoamylbutyrat, Ethylbutyrat, Vanillin, Ethyvanillin, Hexanol, Hexanal, cis-3-Hexenol, cis-3-Hexenylacetat, Linalool, alpha-Terpineol, cis- und trans-Carvylacetat, p-Cymol, Damascenon, Damascone, Rosenoxid, Dimethylsulfid, Fenchol, Acetaldehyddiethylacetal, cis-4-Heptenal, Isobutyraldehyd, Isovaleraldehyd, cis-Jasmon, Anisaldehyd, Methylsalicylat, Myrtenylacetat, 2-Phenylethylalkohol, 2-Phenylethylisobutyrat, 2-Phenylethylisovalerat, Zimtaldehyd, Geraniol, Nerol. Bei chiralen Verbindungen können die Aromastoffe als Racemat oder als einzelnes Enantiomer oder als enantiomerenangereichertes Gemisch vorliegen.

Beispiele für weitere Aroma- und/oder Geschmacksstoffe, die vorteilhaft mit den erfindungsgemäßen Tetrahydropyranen kombiniert werden können, sind Stoffe mit einer physiologischen Kühlwirkung, d.h. Stoffe, die in den Schleimhäuten eine Kältempfindung hervorrufen. Solche Kühlwirkstoffe sind insbesondere I-Menthol, I-Isopulegol, Menthonacetale (z.B. Menthonglycerinacetal), Menthylester, Ester aus Menthol und Hydroxycarbonsäuren mit 2 bis 6 C-Atomen (z.B. Menthyllactat), substituierte Menthan-3-carbonsäureamide (z.B. Menthan-3-carbonsäure-N-ethylamid), verzweigte Alkancarbonsäureamide (z.B. 2-Isopropyl-N,2,3-trimethylbutanamid), 3,3,5-Trimethylcyclohexanol, 3-Menthoxy-1,2-propandiol, 3-Menthoxy-2-methyl-1,2-propandiol, 2-Menthoxyethanol, 2-Menthoxypropanol, 3-Menthoxypropanol, 4-Menthoxybutanol, 2-Hydroxyethylmenthylcarbonat, 2-Hydroxypropylmenthylcarbonat, Glycerinmenthylcarbonat, N-Acetylglycinmenthylester, Menthylhydroxycarbonsäureester (z.B. Menthyl-3-hydroxybutyrat), Menthan-3,8-diol, Menthyl-2-methoxyacetat, Menthyl-2-(2-methoxyethoxy)acetat, Menthylmonosuccinat, 2-Mercaptocyclodecanon, Menthyl-2-pyrrolidin-5-oncarboxylat.

Weitere Beispiele für Stoffe mit einer physiologischen Kühlwirkung, d.h. Stoffe, die in den Schleimhäuten eine Kältempfindung hervorrufen, die vorteilhaft mit den erfindungsgemäßen Tetrahydropyranen kombiniert werden können, finden sich in WO 2008/138162 A1 in einer tabellarischen Übersicht. Aus diesen besonders bevorzugt sind aromatische Menthancarbonsäureamide, wie beispielsweise WS-12 [N-(4-Methoxyphenyl)-menthancarbonsäureamid] und Evercool 180 [N-(4-Cyanomethylphenyl)-p-menthanecarboxamide], sowie (1R,2S,5R)-2-isopropyl-5-methyl-N-(2-(pyridin-2-yl)ethyl)cyclohexanecarboxamide.

Bevorzugte Stoffe mit einer physiologischen Kühlwirkung sind Menthylester, Menthonacetale, Menthan-3-carbonsäureamide und verzweigte Alkancarbonsäureamide.

Desweiteren bevorzugte Stoffe mit einer physiologischen Kühlwirkung sind Menthyloxamat, Menthyl-N-methyloxamat, Menthyl-N,N-dimethyloxamat, Menthyl-N-ethyloxamat, Menthyl-N,N-diethyloxamat, Menthyl-N-propyloxamat, Menthyl-N,N-dipropyloxamat, Menthyl-N-isopropyloxamat, Menthyl-N,N-diisopropyloxamat, Menthyl-N-cyclopropyloxamat, Menthyl-N-butyloxamat, Morpholin-4-yl-oxo-essigsäure-(1R,2S,5R)-2-isopropyl-5-methyl-cyclohexylester, Menthyl-N-(2-methoxyethyl)-oxamat, Menthyl-N-(3-methoxypropyl)-oxamat, Menthyl-N-(2-hydroxyethyl)-oxamat, Menthyl-N-(3-hydroxypropyl)-oxamat.

In erfindungsgemäßen Mischungen sind einer oder mehrere der Aromastoffe ohne physiologische Kühlwirkung ausgewählt sind aus der Gruppe bestehend aus Stoffen, die einen scharfen Geschmack oder eine Wärme- oder Hitzempfindung auf Haut und Schleimhäuten oder ein Prickel-, bzw. Kribbelgefühl im Mund- und Rachenraum hervorrufen, insbesondere Paprikapulver, Chili-Pfeffer-Pulver, Extrakte aus Paprika, Extrakte aus Pfeffer, Extrakte aus Chili-Pfeffer, Extrakte aus Ingwerwurzeln, Extrakte aus Paradieskörnern (Aframomum melegueta), Extrakte aus Parakresse (Jambu-Oleoresin; Spilanthes acmella, bzw. Spilanthes oleracea), Extrakte aus Japanischem Pfeffer (Zanthoxylum piperitum), Extrakte aus Kaempferia galanga, Extrakte aus Alpinia galanga, Extrakte aus Wasserpfeffer (Polygonium hydropiper), Capsaicin, Dihydrocapsaicin, Gingerol, Paradol, Shogaol, Piperin, Saanshool-I, Saanshool-II, Sanshoamid, Spilanthol, Carbonsäure-N-Vanillylamide, insbesonders Nonansäure-N-vanillylamid, 2-Nonensäureamide, insbesonders 2-Nonensäure-N-isobutylamid, 2-Nonensäure-N-4-hydroxy-3-methoxyphenylamid, 2,4-Decadiensäureamide, vorzugsweise 2,4-Decadiensäure-N-isobutylamide, insbesondere Pellitorine gemäß WO 2004/000787 bzw. US 2004/0241312, insbesondere 2E,4Z-Decadiensäure-N-isobutylamid (cis-Pellitorin) und 2E,4E-Decadiensäure-N-isobutylamid (trans-Pellitorin) sowie deren Mischungen, Alkencarbonsäure-N-alkylamide gemäß DE 103 51 422, Alkylether von 4-Hydroxy-3-methoxybenzylalkohol, insbesonders 4-Hydroxy-3-methoxybenzyl-n-butylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, Acetale von Vanillin, Acetale von Ethylvanillin, Acetale von Isovanillin, (4-Hydroxy-3-methoxyphenyl)essigsäureamide, insbesonders (4-Hydroxy-3-methoxyphenyl)essigsäure-N-n-octylamid, Allylisothiocyanat, Nicotinaldehyd, Methylnicotinat, Propylnicotinat, 2-Butoxyethylnicotinat, Benzylnicotinat und 1-Acetoxychavicol.

In Aromakompositionen beträgt die eingesetzte Gesamtmenge der erfindungsgemäßen alkylierten Tetrahydropyrane vorzugsweise 0,5 bis 30 Gew.-%, vorzugsweise 1 bis 20 Gew.-% und besonders bevorzugt 2 bis 10 Gew.-%, bezogen auf das Gesamtgewicht der Aromakomposition.

Erfindungsgemäß angegeben wird ferner eine der Ernährung, der Mundhygiene oder dem Genuss dienende Zubereitung oder pharmazeutische oder kosmetische Zubereitung, umfassend (i) ein oder mehrere erfindungsgemäße alkylierte Tetrahydropyrane oder (ii) eine erfindungsgemäße Mischung, wobei der Bestandteil (i) oder, wenn vorhanden, (ii) vorliegt in einer ausreichenden Konzentration
- zum Erreichen einer physiologischen Kühlwirkung auf der Haut und/oder einer Schleimhaut und/oder
- zum Vermitteln, Modifizieren oder Verstärken eines Frischegefühls in Mund, Rachen und/oder den Atemwegen.

Die erfindungsgemäßen alkylierten Tetrahydropyrane liegen (vorzugsweise als Bestandteil einer Aromakompositionen in den oben angegebenen Mengenbereichen), je nach Zubereitungsart, allgemein in solchen Zubereitungen vorzugsweise in einem Gesamtanteil von 10 ppm bis 2 Gew.-% vor. Bevorzugt ist ein Gehalt von 25 ppm bis 1 Gew.-%; besonders bevorzugt ein Gehalt von 50 ppm bis 0,5 Gew.-%, am meisten bevorzugt ein Gehalt von 100 ppm - 0,25 Gew.-%, jeweils bezogen auf die gesamte Zubereitung. Zu bestimmten besonders bevorzugten Zubereitungen, insbesondere Mundhygieneprodukten, werden nachfolgend weitere bevorzugte Gehalte genannt.

Die die erfindungsgemäßen Verbindungen enthaltenden Aroma- oder Geschmacksstoffkompositionen können in reiner Form, als Lösungen oder auch in besonders zubereiteter Form verwendet und in gebrauchsfertige Produkte eingearbeitet werden.

Als Lösungsmittel eignen sich z.B. Ethylalkohol, 1,2-Propylenglycol, Glycerin, Triacetin, Benzylakohol und fette Öle wie z.B. Kokosöl oder Sonnenblumenöl.

Die die erfindungsgemäßen Verbindungen enthaltenden Zubereitungen können auch Zusatz- und Hilfsstoffe,insbesondere Konservierungsstoffe, Farbstoffe, Antioxidantien, Fließmittel, Verdickungsmittel, etc. enthalten.

Die erfindungsgemäßen Verbindungen können an einen Träger gebunden, sprühgetrocknet oder auch verkapselt vorliegen. In der gebundenen Form können erfindungsgemäßen Verbindungen an oder in einen Träger gebunden sein, z.B. Kochsalz, Zucker, Stärken oder Zuckerschmelzen. Die sprühgetrocknete Form wird aus den flüssigen Kompositionen hergestellt, indem man eine Emulsion unter Zugabe von bestimmten Mengen eines Trägerstoffs, vorzugsweise Biopolymere wie Stärke, modizierte Stärken, Maltodextrin und Gummi Arabicum, herstellt. Diese Emulsion wird in Sprühtrocknern durch Feinstverteilung bei gleichzeitiger Temperaturanwendung getrocknet. Es resultiert ein Pulver mit der gewünschten Beladung an flüssiger Komposition. Die verkapselte Form wird ebenfalls aus den flüssigen Kompositionen durch Zugabe eines Trägerstoffs hergestellt. Es gibt verschiedenen Technologien, mit denen Kapseln hergestellt werden können. Die gängigsten sind die Extrusion, die Sprühgranulation und die Coazervation. Die Partikelgrößen reichen üblicherweise von 10 µm bis 5 mm. Die gängigsten Kapselmaterialien sind verschiedene Stärken, Maltodextrin und Gelatine. In diesen Kapseln sind die flüssigen oder festen Aroma- oder Geschmacksstoffkompositionen eingeschlossen und können durch verschiedene Mechanismen wie Wärmeanwendung, pH-Verschiebung oder Kaudruck freigesetzt werden.

Die die erfindungsgemäßen Verbindungen enthaltenden Zubereitungen können vorteilhaft vor allem in Mundhygieneprodukten, wie Zahnpasten und Mundwässern, Kaugummis, Nahrungsmitteln, wie Süßwaren und Lutschbonbons, Genußmitteln wie Tabak und pharmazeutischen Präparaten und Nasensprays eingesetzt werden.

Der Gesamtgehalt einer Aromakomposition enthaltend eine oder mehrere erfindungsgemäße Verbindungen beträgt in gebrauchsfertigen Mundwässern vorzugsweise 0,01 bis 1 Gew.%, bevorzugt 0,05 bis 0,5 Gew.-%, besonders bevorzugt ist ein Gehalt von 0,1 bis 0,3 Gew.-%, jeweils bezogen auf das gesamte Mundwasser.

In Mundwasserkonzentraten beträgt der Gesamtgehalt der Aromakomposition enthaltend eine oder mehrere erfindungsgemäße Verbindungen vorzugsweise 0,1 bis 15 Gew.-%, bevorzugt ist ein Gehalt von 0,5 bis 8 Gew.-%, besonders bevorzugt 1 bis 5 Gew.-%, jeweils bezogen auf das gesamte Mundwasserkonzentrat.

In Zahnpasten beträgt der Gesamtgehalt der Aromakomposition enthaltend eine oder mehrere erfindungsgemäße Verbindungen vorzugsweise 0,1 bis 5 Gew.-%, bevorzugt 0,5 bis 2 Gew.-%, besonders bevorzugt 0,8 bis 1,5 Gew.-%, jeweils bezogen auf die gesamte Zahnpasta.

In Kaugummis beträgt der Gesamtgehalt der Aromakomposition enthaltend eine oder mehrere erfindungsgemäße Verbindungen vorzugsweise 0,1 bis 5 Gew.-%, bevorzugt 0,5 bis 3 Gew.-%, besonders bevorzugt 0,8 bis 2,5 Gew.-%, jeweils bezogen auf das gesamte Kaugummi.

In Lutschbonbons (Hartkaramellen) beträgt der Gesamtgehalt der Aromakomposition enthaltend eine oder mehrere erfindungsgemäße Verbindungen vorzugsweise 0,01 bis 2 Gew.-%, bevorzugt 0,05 bis 1 Gew.-%, besonders bevorzugt 0,1 bis 0,5 Gew.-%, jeweils bezogen auf das gesamte Lutschbonbon.

### Bevorzugt ist die erfindungsgemäße Zubereitung

(i) eine der Ernährung oder dem Genuss dienende Zubereitung ausgewählt aus Backwaren, Süßwaren, alkoholische oder nicht-alkoholische Getränke, Instantgetränke, Fleischprodukte, Eier oder Eiprodukte , Getreideprodukte, Milchprodukte, Fruchtzubereitungen, Gemüsezubereitungen, Knabberartikel, Produkte auf Fett- und Ölbasis oder Emulsionen derselben, sonstige Fertiggerichte und Suppen, Gewürze, Würzmischungen, Aufstreuwürzungen, Halbfertigwaren, Nahrungsergänzungsmittel; oder
(ii) eine der Mundhygiene dienende Zubereitung, vorzugsweise auf Basis eines Zahnpflegemittels, und ausgewählt aus der Gruppe bestehend aus: Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide und Zahnpflegekaugummi; oder
(iii) eine pharmazeutische Zubereitung, wobei die Zubereitung vorzugsweise eine orale pharmazeutische Zubereitung oder eine nasal zu applizierende Zubereitung ist, vorzugsweise in Form von Kapseln, Tabletten, Dragees, Granulaten, Pellets, Feststoffmischungen, Dispersionen in flüssigen Phasen, als Emulsionen, als Pulver, als Lösungen, als Pasten oder als andere schluck- oder kaubare Zubereitung; oder
(iv) eine kosmetische Zubereitung, ausgewählt aus der Gruppe bestehend aus: Seife, Syndet, flüssiges Wasch-, Dusch-, oder Badepräparat, Emulsion, Salbe, Paste, Gel, Öl, Toner, Balsam, Serum, Puder, Eau de Toilette, Toilette, Eau de Cologne, Parfum, Wachs, Stift, Roll-On, (Pump-)Spray, Aerosol (schäumend, nicht schäumend oder nachschäumend), Fußpflegemittel, Bartreinigungs- oder -pflegemittel, Insekten abwehrendes Mittel, Sonnenschutzmittel, Aftersun-Präparat, Rasiermittel, After-Shave, Haarentfernungsmittel, Haarpflegemittel, Conditioner, Haarkur, Haarwasser, Haarspülung, Frisiercreme, Pomade, Dauerwell- und Fixierungsmittel, Haarglättungsmittel, Haarfestiger, Styling-Hilfe, Blondiermittel, Haaraufheller, Haarconditioner, Haarmousse, Haartönung, Nagelpflegemittel, Deodorant, Antitranspirant, Mundwasser, Munddusche, Make-Up, Make-Up-Entferner, Augenpflege, Lippenkosmetika, Lippenpflegemittel, dekorative Kosmetik, Badeartikel und Maske.

Der Mundhygiene dienende Zubereitungen, insbesondere Zahnpasten, die mit den die erfindungsgemäßen Verbindungen enthaltenden Kompositionen aromatisiert werden, bestehen im allgemeinen aus einem abrasiven System (Schleif- oder Poliermittel), wie z.B. Kieselsäuren, Calciumcarbonaten, Calciumphosphaten, Alumiuniumoxiden und/oder Hydroxylapatiten, aus oberflächenaktiven Substanzen, wie z.B. Natriumlaurylsulfat, Natriumlaurylsarcosinat und/oder Cocamidopropylbetain, aus Feuchthaltemitteln, wie z.B. Glycerin und/oder Sorbit, aus Verdickungsmitteln, wie z.B. Carboxymethylcellulose, Polyethylenglycolen, Carrageenanen und/oder Laponiten^{®}, aus Süßstoffen, wie z.B. Saccharin, aus Kühlwirkstoffen, aus Stabilisatoren und aus aktiven Wirkstoffen, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, und/oder Natriumbicarbonat.

Kaugummis, die mit den die erfindungsgemäßen Verbindungen enthaltenden Kompositionen aromatisiert werden, umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren und Stabilisatoren.

Im Stand der Technik sind zahlreiche unterschiedliche Kaugummibasen bekannt, wobei zwischen sogenannten "chewing gum" - oder um "bubble gum" - Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerin-monostearat.

Bei der Anwendung von Fertigprodukten, die Kompositionen mit den erfindungsgemäßen Verbindungen enthalten, zeigt es sich, dass die erfindungsgemäßen Verbindungen, bzw. die die erfindungsgemäßen Verbindungen enthaltenden Kompositionen sich besonders dafür eignen, die Atemluft zu erfrischen und schlechten Mundgeruch zu neutralisieren, bzw. zu reduzieren.

Die Verwendung der erfindungsgemäßen Verbindungen, bzw. der die erfindungsgemäßen Verbindungen enthaltenden Mischungen in Mundpflegeprodukten (Mundhygieneprodukten), wie z. B. Mundwässern und Zahnpasten, und Kaugummis führt dazu, dass unangenehme, vor allem bittere oder metallische Geschmackseindrücke teilweise maskiert oder neutralisiert werden, die z.B. durch Stoffe wie Triclosan, Zinkcitrat, -sulfat, Poly- und Pyrophosphaten, Bicarbonate, Strontium- und Kaliumsalze, Zinnpyrophosphat, - chlorid, Aluminiumlactat, Wasserstoffperoxid, Fluoride, Vitamine, Cetylpyridiniumchlorid sowie von Emulgatoren, wie z.B. besonders Natriumlaurylsulfat, Natriumlaurylsarkosinat und Cocamidopropylbetain, und Süßstoffen, wie z.B. Aspartam, Saccharin, Acesulfam-K, Sorbit; Xylit, Cyclamate (z.B. Natriumcyclamat), Sucralose, Alitam, Neotam, Thaumatin, Neohesperidin Dihydrochalcon, Maltit, Lactit oder Kaugummi-Massen hervorgerufen werden.

Als weitere positive Eigenschaft der erfindungsgemäßen Verbindungen ist ihre Stabilität in Zahnpasten auf Kreide- oder Bicarbonatbasis hervorzuheben, die wegen ihres alkalischen pH-Wertes schwierig zu aromatisieren sind.

Die erfindungsgemäßen Verbindungen, bzw. die die erfindungsgemäßen Verbindungen enthaltenden Kompositionen eignen sich jedoch auch für den Einsatz in pharmazeutischen Präparaten, wie z. B. Nasentropfen und -sprays oder Einreibepräparaten. Insbesondere eignen sich die die erfindungsgemäßen Verbindungen enthaltenden Kompositionen für die Maskierung des bitteren Geschmacks von Medikamenten.

Weitere Aspekte der vorliegenden Erfindung ergeben sich aus den nachfolgenden Beispielen sowie der beigefügten Patentansprüchen. Sofern nicht anders angegeben, beziehen sich alle Angaben auf das Gewicht.

In manchen Beispielen wurde eine Pellitorin-Lösung (im Folgenden als "Pellitorin Lösung PLM" bezeichnet) eingesetzt bestehend aus 10% Pellitorin (umfassend 4,9% 2E,4Z-Decadiensäure-N-isobutylamid und 94,3% 2E,4E-Decadiensäure-N-isobutylamid), 45% Propylenglycol und 45% natürliches Pfefferminzöl (*Mentha arvensis*).

### Beispiele:

### Beispiel 1.0: Panel untersuchung einer Zahnpastaprobe (13 sensorisch geschulte Probanden):

Es wurden zwei Zahnpastaproben [Zahnpastaformulierung I aus Beispiel 2.15 ("Silica Opaque"), Aroma aus Beispiel 2.1 ("Pfefferminztyp")] gegeneinander verglichen. Die Probanden waren aufgefordert sich eine Minute lang die Zähne zu putzen und anschliessend die Proben sensorisch zu bewerten. Abgefragt wurden die unten im Netzdiagramm aufgeführten Deskriptoren (Skala von 0 (= entspricht nicht dem Deskriptor) bis 8 (entspricht sehr stark dem Deskriptor)).

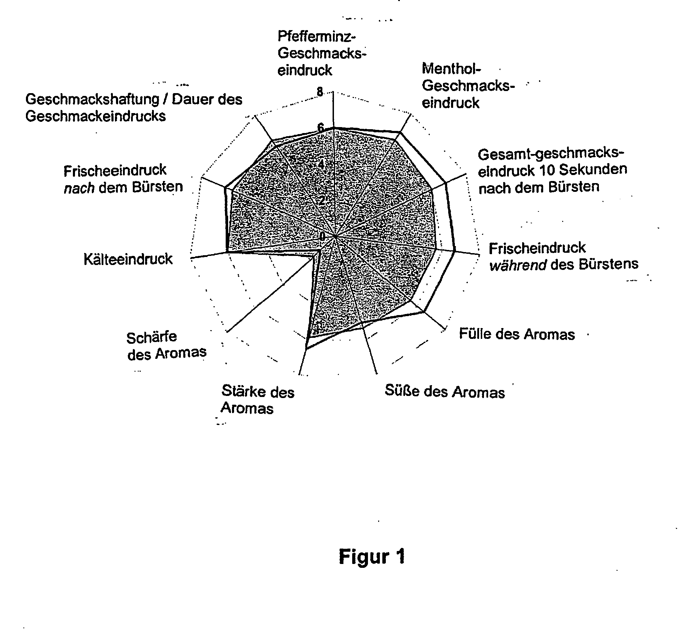

Eine Probe enthielt das Aroma "Pfefferminztyp" ohne erfindungsgemäße Verbindungen (Dosierung 0,9 %; ausgefüllter Bereich der Figur 1) und die Vergleichsprobe enthielt das Aroma aus Beispiel 2.1 (Dosierung 0,8 %; durchgezogene Linie im Diagramm 1) mit den erfindungsgemäßen Verbindungen.

Die erfindungsgemäßen Verbindungen verändern demnach nicht das Geschmacksprofil des Aromas; vielmehr verstärken sie dessen Geschmackseindruck in gewünschter Art und Weise. Der Eindruck von Frische und Fülle wird verstärkt und die Gesamtwirkung des Aromas entfaltet sich besser. Insbesondere in der Anfangsphase des Bürstens (beim Aufschäumen der Paste) wird der Geschmackseindruck deutlich verstärkt (Deskriptoren "Freshness during" (Frische während des Putzens) und "Impact" (Gesamtgeschmackseindruck 10 Sekunden nach dem Putzen). Von 13 Probanden bevorzugten 9 das Muster, welches die erfindungsgemäßen Verbindungen enthielt (vgl. Figur 1).

| **Deskriptor** | **Bedeutung** |
|---|---|
| "Peppermint" | Pfefferminz-Geschmackseindruck |
| "Menthol" | Menthol-Geschmackseindruck |
| "Impact" | Gesamtgeschmackseindruck 10 Sekunden nach dem Bürsten |
| "Freshness during" | Frischeindruck *während* des Bürstens |
| "Fullness" | Fülle des Aromas |
| "Sweetness" | Süße des Aromas |
| "Strength" | Stärke des Aromas |
| "Sharpness" | Schärfe des Aromas |
| "Cooling" | Kälteeindruck |
| "Freshness after" | Frischeeindruck nach dem Bürsten |
| "Long Lasting Taste" | Geschmackshaftung / Dauer des Geschmackeindrucks |

### Beispiel 1.1: Herstellung verschiedener alkyl- und alkenylsubstituierter Tetrahydropyrane am Beispiel von 2,6-Diethyl-5-isopropyl-2-methyl-tetrahydropyran:

### 1. Stufe:

In einem 2L-Doppelmantelreaktor mit Thermometer und Tropftrichter wurden 46 g Wasser vorlegt und 346 g Phosphorsäure (85%ig) zugegeben (also auf 75 %ige Konzentration verdünnt) und anschließend auf 0°C gekühlt.
116,2 g Propionaldehyd mit 308,5 g Linalool wurden gemischt und unter starkem Rühren in ca. 1h bis zu einer maximalen Innentemperatur von 5°C zugetropft. Den Ansatz wurde nach beendeter Zugabe weitere 4 h bei 0°C bis 5°C nachgerührt, dann auf Raumtemperatur (ca. 20°C) erwärmt. Anschließend wurde zur vollständigen Umsetzung für etwas 20 Minuten auf 70 °C aufgeheizt. Bei 65 - 70°C wurde die Phasen getrennt. Die organische Phase wurde zweimal mit jeweils 100 g Wasser gewaschen und anschließend der Rohansatz über eine 30 cm Füllkörperkolonne destilliert. Es wurden 251 g Produkt mit einerm Gehalt von 84% an 6-Ethyl-5-isopropenyl-2-methyl-2-vinyltetrahydropyran erhalten (54% Ausbeute der Theorie).

### 2. Stufe:

Die Hydrierung wurde in einem Hydrierautoklaven durchgeführt. Es wurden 202 g 6-Ethyl-5-isopropenyl-2-methyl-2-vinyltetrahydropyran (aus der 1. Stufe) mit 8 g Pd/Aktivkohle (Pd-Gehalt: 5%) in den Autoklaven gefüllt und bei 30 bar Wasserstoffdruck hydriert. Die Reaktion verlief exotherm von 20°C bis etwa 50°C, danach wurde auf 90°C geheizt. Die H₂-Aufnahme war nach 35 min beendet. Es wurden 107% H₂ derTheorie aufgenommen. Der Katalysator wurde abfiltriert und dieser anschließend mit Ethanol nachgespült. Das so erhaltene Material wurde anschließend über 120 cm Kolonne destilliert. Es wurden 131 g 2,6-Diethyl-5-isopropyl-2-methyl-tetrahydropyran (Reinheit > 97 %) erhalten (73% Ausbeute der Theorie).

### 6-Ethyl-5-isopropenyl-2-methyl-2-vinyltetrahydropyran

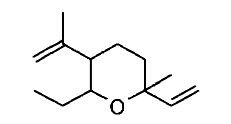

**¹H-NMR** (Mischung der Isomeren, 400 MHz, CDCl₃): δ = 0.92 (t, J = 7.4 Hz, 3 H), 1.18-1.31 (m, 2 H), 1.19 (s, 0.5 × 3 H), 1.28 (s, 0.5 × 3 H), 1.45-1.95 (m, 6 H), 1.62 (dd, J = 0.9 Hz,J=1.5Hz,0.5×3H), 1.70 (dd, J = 1.0 Hz, J = 1.4 Hz, 0.5 × 3 H), 3.45 (ddd, J = 2.6 Hz, J=8.7Hz, J= 10.2 Hz, 0.5 × 1 H), 3.53 (ddd, J = 2.6 Hz, J = 8.2 Hz, J = 9.7 Hz, 0.5 × 1 H), 4.68-4.76 (m, 1 H), 4.89-5.25 (m, 1 H), 5.79 (ddd, J = 1.0 Hz, J = 11.1 Hz, J = 17.8 Hz, 1 H), 5.91 (dd, J = 10.9 Hz, J = 17.4 Hz, 1 H) ppm.
**¹³C-NMR** (Mischung der Isomeren, 100 MHz, CDCl₃): δ = 10.18 (CH₃), 10.33 (CH₃), 20.26 (CH₃), 20.39 (CH₃), 21.03 (CH₃), 27.28 (CH₂), 27.44 (CH₂), 27.60 (CH₂), 27.96 (CH₂), 27.99 (CH₂), 31.55 (CH₃), 35.52 (CH₂), 35.92 (CH₂), 50.87 (CH), 50.96 (CH), 74.38 (C), 74.69 (CH), 76.04 (C), 76.17 (CH), 111.04 (CH₂), 112.17 (CH₂), 112.21 (CH₂), 115.34 (CH₂), 144.42 (CH), 147.77 (CH), 148.69 (C) ppm.
**MS:** *m*/*z* (%) = 194 (1) [M⁺], 136 (10), 121 (19), 107 (14), 93 (32), 81 (11), 68 (100), 53 (8).

### 2,6-Diethyl-5-isopropyl-2-methyl-tetrahydropyran

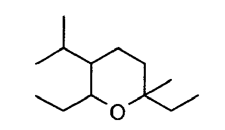

**¹H-NMR** (Mischung der Isomeren, 400 MHz, CDCl₃): δ = 0.74-0.96 (m, 12 H), 1.09 (s, 0.5 × 3 H), 1.10 (s, 0.5 × 3 H), 1.10-1.72 (m, 8 H), 1.81-1.93 (m, 2 H), 3.22 (ddd, J = 1.0 Hz, J = 2.7 Hz, J = 8.4 Hz, 0.5 ×1 H), 3.33 (ddd, J = 2.8 Hz, J = 8.2 Hz, J = 10.1 Hz, 0.5 ×1 H) ppm.
**¹³C-NMR** (Mischung der Isomeren, 100 MHz, CDCl₃): δ = 7.38 (CH₃), 7.72 (CH₃), 9.59 (CH₃), 9.87 (CH₃), 15.86 (CH₃), 15.88 (CH₃), 18.07 (CH₂), 18.32 (CH₂), 19.39 (CH₃), 21.33 (CH₃), 25.66 (CH₂), 25.78 (CH₂), 25.84 (CH₂), 26.86 (CH), 26.87 (CH), 27.66 (CH₃), 34.89 (CH₂), 36.19 (CH₂), 37.17 (CH₂), 44.70 (CH), 45.13 (CH), 72.50 (C), 72.51 (C), 72.83 (CH), 73.03 (CH) ppm.
**MS:** *m*/*z* (%) = 198 (1) [M⁺], 169 (66), 151 (79), 111 (25), 109 (21), 95 (48), 83 (18), 70 (100), 55 (57).

### Beispiel 1.2: Herstellung verschiedener alkyl- und alkenylsubstituierter Tetrahydropyrane am Beispiel von 2,2,6-Trimethyl-5-isopropyltetrahydropyran

In einem 2L-Dreihalskolben wurden 42 g Acetaldehyd in 600 mL Diethylether vorgelegt und auf 0°C abgekühlt. Anschließend wurden 140 g einer 48%igen Lösung von Bortrifluorid-Diethyletherat zugegeben und 10 Minuten bei 0°C gerührt. Zur Reaktionslösung wurden bei 0°C 124 g 2,6-Dimethyl-hept-5-en-2-ol in 100 mL Diethylether gelöst zugetropft. Nach vollendeter Zugabe wurde noch eine Stunde bei 0°C nachgerührt und anschließend auf Raumtemperatur erwärmt. Nachdem die Reaktionslösung 12 Stunden bei Raumtemperatur gerührt worden war, wurde mit gesättigter Ammoniumchloridlösung (500 mL) gequencht und nach Aufarbeitung das erhaltene Rohprodukt über eine 30 cm Füllkörperkolonne destilliert. Es wurden 82 g 2,2,6-Trimethyl-5-isopropenyltetrahydropyran in 98%iger Reinheit erhalten. (55% Ausbeute der Theorie).
Das 2,2,6-Trimethyl-5-isopropyltetrahydropyran wurde durch Hydrierung (analog der 2. Stufe aus Beispiel 1.1) von 2,2,6-Trimethyl-5-isopropenyltetrahydropyran und anschließender Destillation in 85%iger Ausbeute erhalten.

### 2,2,6-Trimethyl-5-isopropyltetrahydropyran

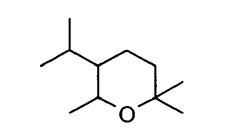

**¹H-NMR** (400 MHz, CDCl₃): δ = 0.79 (d, J = 6.9 Hz, 3 H), 0.91 (d, J = 7.0 Hz, 3 H), 1.00-1.10 (m, 1 H), 1.11-1.60 (m, 4 H), 1.14 (d, J=6.1 Hz, 3 H), 1.18 (s, 3 H), 1.20 (s, 3 H), 1.87 (dhept., J = 3.2 Hz, J = 6.9 Hz, 1 H), 3.56 (dq, J = 6.1 Hz, J = 10.1 Hz, 1 H) ppm.
**¹³C-NMR** (100 MHz, CDCl₃): = 15.79 (CH₃), 18.44 (CH₂), 20.05 (CH₃), 21.31 (CH₃), 22.08 (CH₃), 27.25 (CH), 31.74 (CH₃), 36.86 (CH₂), 47.59 (CH), 69.15 (CH), 71.36 (C) ppm.
**MS:** *m*/*z* (%) = 170 (1) [M⁺], 155 (21), 137 (8), 126 (22), 111 (3), 97 (5), 84 (22), 69 (36), 56 (100), 43 (30).

### In Anlehnung an die Beispiele 1.1 und 1.2 wurden unter anderen folgende Verbindungen der Formel (I) hergestellt:

### 2,6-Dimethyl-5-isopropyl-2-thyltetrahydropyran

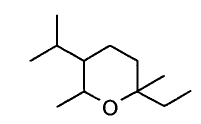

**¹H-NMR** (400 MHz, CDCl₃): δ = 0.77 (d, J = 6.9 Hz, 3 H), 0.78-0.90 (m, 1 H), 0.83 (t, J = 7.48 Hz, 3 H), 0.90 (d, J = 6.9 Hz, 3 H), 1.03-1.13 (m, 1 H), 1.10 (s, 3 H), 1.13 (d, J = 6.12 Hz, 3 H), 1.25-1.48 (m, 4 H), 1.55-1.61 (m, 1 H), 1.79-1.89 (m, 1 H), 3.44 (dq, J = 6.1 Hz, J = 10.0 Hz, 1 H) ppm.
**¹³C-NMR** (100 MHz, CDCl₃): δ = 7.59 (CH₃), 15.83 (CH₃), 18.12 (CH₂), 20.04 (CH₃), 21.31 (CH₃), 25.93 (CH₂), 27.30 (CH), 27.80 (CH₃), 35.90 (CH₂), 47.31 (CH), 68.35 (CH), 72.89 (C) ppm.
**MS:** *m*/*z* (%) = 185 (1) [MH⁺], 169 (1), 155 (79), 137 (100), 111 (6), 97 (43), 81 (24), 70 (72), 55 (59).

### 2,6-Dimethyl-5-isopropenyl-2-vinyltetrahydropyran

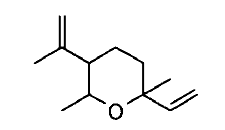

**¹H-NMR** (Mischung der Isomeren, 400 MHz, CDCl₃): δ = 1.08 (d, J = 6.2 Hz, 3 H), 1.10 (d, J = 6.1 Hz, 3 H), 1.23 (s, 3 H), 1.32 (s, 3 H), 1.50-1.92 (m, 12 H), 1.63 (dd, J = 1.0 Hz, J=1.4Hz, 3H), 1.70 (dd, J = 0.9 Hz, J = 1.4 Hz, 3 H), 3.62 (dq, J = 6.1 Hz, J = 9.9 Hz, 1 H), 3.72 (dq, J = 6.1 Hz, J = 9.7 Hz, 1 H), 4.70-4.77 (m, 2 H), 4.85-5.23 (m, 2 H), 5.80 (ddd, J = 1.1 Hz, J = 11.0 Hz, J = 5.8 Hz, 2 H), 5.94 (dd, J = 10.8 Hz, J = 17.4 Hz, 2 H) ppm.
**¹³C-NMR** (Mischung der Isomeren, 100 MHz, CDCl₃): δ = 20.03 (CH₃), 20.18 (CH₃), 20.28 (CH₃), 20.34 (CH₃), 25.96 (CH₂), 26.54 (CH₂), 31.18 (CH₃), 34.29 (CH₂), 34.74 (CH₂), 51.47 (CH), 51.56 (CH), 68.77 (CH), 70.09 (CH), 73.37 (C), 74.92 (C), 111.16 (CH₂), 111.47 (CH₂), 111.57 (CH₂), 114.61 (CH₂), 143.17 (CH), 146.36 (CH), 147.30 (C), 147.40 (C) ppm.
**MS:** *m*/*z* (%) = 180 (1) [M⁺], 165 (1), 136 (8), 121 (18), 107 (16), 93 (35), 79 (11), 68 (100), 53 (16).

### 6-Isobutyl-5-isopropenyl-2,2-dimethyltetrahydropyran

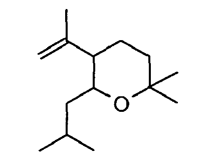

**¹H-NMR** (400 MHz, CDCl₃): δ = 0.83 (d, J = 6.6 Hz, 3 H), 0.87 (d, J = 6.8 Hz, 3 H), 1.11-1.29 (m, 2 H), 1.20 (s, 3 H), 1.21 (s, 3 H), 1.42-1.58 (m, 2 H), 1.67 (dd, J = 0.9 Hz, J = 1.4 Hz, 3 H), 1.68-1.88 (m, 4 H), 3.54 (dt, J = 2.5 Hz, J = 9.9 Hz, 1 H), 4.72 (s, br., 2 H) ppm. **¹³C-NMR** (100 MHz, CDCl₃): δ = 20.10 (CH₃), 21.14 (CH₃), 21.89 (CH₃), 23.81 (CH), 23.92 (CH₃), 26.69 (CH₂), 31.75 (CH₃), 36.38 (CH₂), 42.76 (CH₂), 50.50 (CH), 69.95 (CH), 71.08 (C), 111.51 (CH₂), 147.55 (C) ppm.
**MS:** *m*/*z* (%) = 210 (1) [M⁺], 153 (1), 124 (22), 109 (32),95 (6), 81 (10), 68 (100), 57 (8), 41 (21).

### Beispiel 2: Aromakompositionen sowie Formulierunqsbeispiele

### Beispiel 2.1: Aroma Pfefferminztyp

| **Bestandteil** | **Anteil [%]** |
|---|---|
| **2,6-Diethyl-5-isopropyl-2-methyl-tetrahydropyran** | **6** |
| Anethol | 9 |
| I-Menthol (natürlich oder synthetisch) | 35 |
| Pfefferminzöl Piperita Typ (natürlich oder rekonstituiert) | 20 |
| Pfefferminzöl Arvensis Typ (natürlich oder rekonstituiert) | 30 |

### Beispiel 2.2: Aroma Pfefferminztyp Cool

| **Bestandteil** | **Anteil [%]** |
|---|---|
| **2,6-Diethyl-5-isopropyl-2-methyl-tetrahydropyran** | **6** |
| Anethol | 9 |
| I-Menthol (natürlich oder synthetisch) | 35 |
| Pfefferminzöl Piperita Typ (natürlich oder rekonstituiert) | 20 |
| Pfefferminzöl Arvensis Typ (natürlich oder rekonstituiert) | 20 |
| 2-Isopropyl-N,2,3-trimethylbutyramid (WS-23) | 2 |
| (1 R,2S,5R)-N-Ethyl-2-isopropyl-5-methylcyclohexanecarboxamid (WS-3) | 2 |
| Menthol-propyleneglycol-carbonat (Frescolat MPC^{®}) | 2 |
| Menthol-ethylenglycol-carbonat (Frescolat MGC^{®}) | 2 |
| I-Menthyl lactat (Frescolat ML^{®}) | 2 |

### Beispiel 2.3: Aroma Krauseminztyp

| **Bestandteil** | **Anteil [%]** |
|---|---|
| **2,6-Diethyl-5-isopropyl-2-methyl-tetrahydropyran** | **6** |
| Anethol | 9 |
| I-Menthol (natürlich oder synthetisch) | 30 |
| Pfefferminzöl Piperita Typ (natürlich oder rekonstituiert) | 5 |
| Pfefferminzöl Arvensis Typ (natürlich oder rekonstituiert) | 5 |
| I-Carvon | 15 |
| Krauseminzöl Cardiaca Typ (natürlich oder rekonstituiert) | 15 |
| Krauseminzöl spicata Typ (natürlich oder rekonstituiert) | 15 |

### Beispiel 2.4: Aroma Wintergrüntyp

| **Bestandteil** | **Anteil [%]** |
|---|---|
| **2,6-Diethyl-5-isopropyl-2-methyl-tetrahydropyran** | **8** |
| Anethol | 9 |
| I-Menthol (natürlich oder synthetisch) | 45 |
| Pfefferminzöl Piperita Typ (natürlich oder rekonstituiert) | 2 |
| Pfefferminzöl Arvensis Typ (natürlich oder rekonstituiert) | 3 |
| Krauseminzöl Spicata Typ (natürlich oder rekonstituiert) | 1 |
| Eugenol | 7 |
| Eucalyptol | 5 |
| Methylsalicylat | 20 |

### Beispiel 2.5: Aroma Wintergrüntyp Cool

| **Bestandteil** | **Anteil [%]** |
|---|---|
| **2,6-Diethyl-5-isopropyl-2-methyl-tetrahydropyran** | **6** |
| Anethol | 8 |
| 1,2-Propylenglycol | 7 |
| I-Menthol (natürlich oder synthetisch) | Ad 100 |
| Pfefferminzöl Piperita Typ (natürlich oder rekonstituiert) | 2 |
| Pfefferminzöl Arvensis Typ (natürlich oder rekonstituiert) | 3 |
| Krauseminzöl Spicata Typ (natürlich oder rekonstituiert) | 1 |
| Eugenol | 6 |
| Eucalyptol | 4 |
| Methylsalicylat | 16 |
| 2-Isopropyl-N,2,3-trimethylbutyramid (WS-23) | 2 |
| (1*R*,2*S*,5*R*)-N-Ethyl-2-isopropyl-5-methylcyclohexanecarboxamid (WS-3) | 2 |
| Menthol-propyleneglycol-carbonat (Frescolat MPC^{®}) | 2 |
| Menthol-ethylenglycol-carbonat (Frescolat MGC^{®}) | 2 |
| 1-Menthyl lactat (Frescolat ML^{®}) | 2 |
| (1*R*,2*S*,5*R*)-N-[4-(cyanomethyl)phenyl]-2-isopropyl-5- methylcyclohexanecarboxamid | 1 |

### Beispiel 2.6: Aroma Eucalyptustyp

| **Bestandteil** | **Anteil [%]** |
|---|---|
| **2,6-Diethyl-5-isopropyl-2-methyl-tetrahydropyran** | **7** |
| Anethol | 18 |
| Eucalyptol | 15 |
| Eucalyptusöl | 5 |
| I-Menthol (natürlich oder synthetisch) | 50 |
| Pfefferminzöl Piperita Typ (natürlich oder rekonstituiert) | 2 |
| Pfefferminzöl Arvensis Typ (natürliche oder rekonstituiert) | 3 |

### Beispiel 2.7: Aroma Eucalyptustyp Cool

| **Bestandteil** | **Anteil [%]** |
|---|---|
| **2,6-Diethyl-5-isopropyl-2-methyl-tetrahydropyran** | **4** |
| 1,2-Propylenglycol | 5 |
| L-Mentholmethylether | 2 |
| Anethol | 15 |
| Eucalyptol | 15 |
| Eucalyptusöl | 5 |
| I-Menthol (natürlich oder synthetisch) | Ad 100 |
| Pfefferminzöl Piperita Typ (natürlich oder rekonstituiert) | 2 |
| Pfefferminzöl Arvensis Typ (natürlich oder rekonstituiert) | 3 |
| Menthoneglycerinacetal (Frescolat MGA^{®}) | 5 |

### Beispiel 2.8: Aroma Zimttyp

| **Bestandteil** | **Anteil [%]** |
|---|---|
| **2,6-Diethyl-5-isopropyl-2-methyl-tetrahydropyran** | **6** |
| Zimtaldehyd | 10 |
| Anethol | 9 |
| Eugenol | 2 |
| I-Menthol (natürlich oder synthetisch) | 40 |
| Pfefferminzöl Piperita Typ (natürlich oder rekonstituiert) | 10 |
| Pfefferminzöl Arvensis Typ (natürlich oder rekonstituiert) | 15 |
| Krauseminzöl Spicata Typ (natürlich oder rekonstituiert) | 8 |

### Beispiel 2.9: Aroma Zimttyp Cool

| **Bestandteil** | **Anteil [%]** |
|---|---|
| **2,6-Diethyl-5-isopropyl-2-methyl-tetrahydropyran** | **3** |
| Menthylmethylether | 3 |
| Zimtaldehyd | 10 |
| Anethol | 9 |
| Eugenol | 2 |
| I-Menthol (natürlich oder synthetisch) | 40 |
| Pfefferminzöl Piperita Typ (natürlich oder rekonstituiert) | 10 |
| Pfefferminzöl Arvensis Typ (natürlich oder rekonstituiert) | 10 |
| Krauseminzöl Spicata Typ (natürlich oder rekonstituiert) | 8 |
| (1*R*,2*S*,5*R*)-N-Ethyl-2-isopropyl-5-methylcyclohexanecarboxamid (WS-3) | 2 |
| (1*R*,2*S*,5*R*)-N-[4-(cyanomethyl)phenyl]-2-isopropyl-5- methylcyclohexanecarboxamid | 0,5 |
| (1*R*,2*S*,5*R*)-N-[2-(pyridin-2-yl)methyl]-2-isopropyl-5-methylcyclohexanecarboxamid | 0,5 |
| Menthoneglycerinacetal (Frescolat MGA^{®}) | 1 |
| Menthol-propyleneglycol-carbonat (Frescolat MPC^{®}) | 1 |

### Beispiel 2.10: Aroma Typ Eisbonbon

| **Bestandteil** | **I (Gew.%)** | **II (Gew.%)** |
|---|---|---|
| **2,6-Diethyl-5-isopropyl-2-methyl-tetrahydropyran** | **4** | **5** |
| Isoamylacetat | 2 | 2 |
| Ethylbutyrat | 0,5 | - |
| Butylbutyrat | - | 0,5 |
| Ethylvanillin | 2 | - |
| Vanillin | - | 1 |
| Frambinon^{™} [4-(4-Hydroxyphenyl)-2-butanon] | 0,5 | 0,5 |
| I-Menthol (natürlich) | 8 | 11 |
| Triacetin | - | 80 |
| 1,2-Propylenglycol | 83 | - |

### Anwendunqsbeispiele für oben genannte Aromakompositionen:

### Beispiel 2.11: Zuckerfreier Kaugummi mit Aroma Zimttyp Cool

| **Bestandteil** | **I (Gew.-%)** | **II (Gew.-%)** |
|---|---|---|
| Gum Base (Kaugummibase) | 30,00 | 30,00 |
| Sorbit gepulvert | 40,00 | Ad 100 |
| Isomalt gepulvert | 9,50 | 9,50 |
| Xylitol | 2,00 | 2,00 |
| Mannit D | 3,00 | 3,00 |
| Aspartam | 0,10 | 0,10 |
| Acesulfam K | 0,10 | 0,10 |
| Emulgum^{™} (Soja-Lecithine mit hohem Gehalt an Phospholipiden) | 0,30 | 0,30 |
| Sorbitol (70% in Wasser) | 13,00 | 13,00 |
| 1,2-Propylenglyol | - | 1,00 |
| Glycerin | 1,00 | - |
| Pellitorin-Lösung PLM (enthaltend 10% Pellitorin) | - | 0,035 |
| **Aroma Zimttyp Cool (Beispiel 2.9)** | **1,00** | **1,00** |

Bei einer Dosierung von 1% der Aromakomposition im Kaugummi bewirkt das 2,6-Diethyl-5-isopropyl-2-methyl-tetrahydropyran einen deutlichen "Boosteffekt" und vermittelt - im Vergleich zu dem nicht erfindungsgemäßen Aroma - ein verstärktes Frischegefühl, insbesondere in der Anfangsphase des Verzehrs.

### Beispiel 2.12: Standard Kaugummi

| **Bestandteil** | **I (Gew.-%)** | **II (Gew.-%)** |
|---|---|---|
| Gum Base | 21,00 | 21,00 |
| Glucosesirup | 16,50 | 16,50 |
| Glycerin | 0,50 | 0,50 |
| Zucker gepulvert | 60,00 | 60,00 |
| **Aroma Pfefferminztyp (Beispiel 2.1)** | **2,00** | **-** |
| **Aroma Eucalyptustyp Cool (Beispiel 2.7)** | **-** | **2,00** |

Bei einer Dosierung von 2% der Aromakomposition im Kaugummi bewirkt das 2,6-Diethyl-5-isopropyl-2-methyl-tetrahydropyran ein verstärktes Frischegefühl und vermittelt - im Vergleich zu dem nicht erfindungsgemäßen Aroma - eine größere Mundfülle, insbesondere beim Kaubeginn.

### Beispiel 2.13: Zahnpasta (Phosphatbase) mit Aroma Krauseminztyp

| **Bestandteil** | **Anteil [%]** |
|---|---|
| Entionisiertes Wasser | 36,39 |
| Glycerin | 20,00 |
| Solbrol M (Natriumsalz) | 0,15 |
| Natriummonofluorphosphat | 0,76 |
| Saccharin | 0,20 |
| Dicalciumphosphat-Dihydrat | 36,00 |
| Aerosil^{®} 200 (Silica) | 3,00 |
| Natriumcarboxymethylcellulose | 1,20 |
| Natriumlaurylsulfat (Texapon) | 1,30 |
| **Aroma Krauseminztyp (Beispiel 2.3)** | **1,00** |

Bei einer Dosierung von 1% der Aromakomposition in der Zahnpasta bewirkt das 2,6-Diethyl-5-isopropyl-2-methyl-tetrahydropyran bei Bürsten eine Wirkungs-verstärkung des Aromas und vermittelt - im Vergleich zu dem nicht erfindungs-gemäßen Aroma - ein deutliches Frischegefühl, insbesondere beim anfänglichen Bürsten.

### Beispiel 2.14: Zahnpasta (transparente Gelformulierung)

| **Bestandteil** | **I (Gew.%)** | **II (Gew.%)** |
|---|---|---|
| Sorbitol 70% | 63,00 | Ad 100 |
| Entionisiertes Wasser | 11,31 | 11,31 |
| Saccharin | 0,20 | 0,20 |
| Natriummonofluorphosphat | 1,14 | 1,14 |
| Solbrol | 0,15 | 0,15 |
| Trinatriumphosphat | 0,10 | 0,10 |
| PEG 1500 (PEG 32) | 5,00 | 5,00 |
| Sident 9 (Abrasive Silica) | 8,00 | 8,00 |
| Sident 22 S (Verdickende Silica) | 8,00 | 8,00 |
| Natriumcarboxymethylcellulose | 0,60 | 0,60 |
| Natriumlaurylsulfat | 1,50 | 1,50 |
| **Aroma Zimttyp (Beispiel 2.8)** | **1,00** | **-** |
| **Aroma Wintergrüntyp Cool (Beispiel 2.5)** | **-** | **1,00** |
| Pellitorin-Lösung PLM (enthaltend 10% Pellitorin) | - | 0,025 |

Bei einer Dosierung von 1% der Aromakomposition in der Zahnpasta gewinnt die Paste beim Bürsten durch das 2,6-Diethyl-5-isopropyl-2-methyl-tetrahydropyran ein klareres Frischeprofil und vermittelt - im Vergleich zu dem nicht erfindungsgemäßen Aroma - ein eindeutiges retronasales Cooling.

### Beispiel 2.15: Zahnpasta ('Silica opaque')

| **Bestandteil** | **I (Gew.-%)** | **II (Gew.-%)** | **III (Gew.-%)** |
|---|---|---|---|
| Entionisiertes Wasser | 26,53 | 26,53 | 26,53 |
| Sorbitol 70% | 45,00 | Ad 100 | Ad 100 |
| Solbrol M Na-Salz | 0,15 | 0,15 | 0,15 |
| Trinatriumphosphat | 0,10 | 0,10 | 0,10 |
| Saccharin | 0,20 | 0,20 | 0,20 |
| Natriummonofluorphosphat | 1,12 | 1,12 | 1,12 |
| PEG 1500 | 5,00 | 5,00 | 5,00 |
| Sident 9 (Abrasive Silica) | 10,00 | 10,00 | 10,00 |
| Sident 22 S (Dickende Silica) | 8,00 | 8,00 | 8,00 |
| Natriumcarboxymethylcellulose | 0,90 | 0,90 | 0,90 |
| Titan (IV) oxid | 0,50 | 0,50 | 0,50 |
| Natriumlaurylsulfat (SLS) | 1,50 | 1,50 | 1,50 |
| Pellitorin-Lösung PLM (enthaltend 10% Pellitorin) | - | 0,025 | - |
| **Aroma Pfefferminztyp Cool (Beispiel 2.2)** | **1,00** | **1,00** | **-** |
| **Aroma Typ Eisbonbon I (Beispiel 2.10)** | **-** | **-** | **0,70** |

Bei einer Dosierung von 1 % der Aromakomposition in der Zahnpasta gewinnt die Paste beim Bürsten durch das 2,6-Diethyl-5-isopropyl-2-methyl-tetrahydropyran an Mundfülle und erreicht - im Vergleich zu dem nicht erfindungsgemäßen Aroma - ein frischeres Mundgefühl. Zahnpasta III eignet sich insbesondere auch als Zahnpasta für Kinder.

### Beispiel 2.16: Zahnpasta (Calciumcarbonat-Base) mit Aroma Eucalyptustyp Cool

| **Bestandteil** | **I (Gew.%)** | **II (Gew.%)** |
|---|---|---|
| Entionisiertes Wasser | 27,50 | Ad 100 |
| Saccharin | 0,20 | 0,20 |
| Solbrol M Natriumsalz | 0,20 | 0,20 |
| Natriummonofluorphosphat | 0,80 | 0,80 |
| Sorbitol 70% | 29,00 | 29,00 |
| Calciumcarbonat | 35,00 | 35,00 |
| Sident 22 S (Verdickende Silica) | 2,50 | 2,50 |
| Natriumcarboxymethylcellulose | 1,30 | 1,30 |
| Titandioxid | 0,50 | 0,50 |
| Natriumlaurylsulfat | 2,00 | 2,00 |
| **Aroma Eucalyptustyp Cool (Beispiel 2.7)** | **1,00** | **1,00** |
| Pellitorin-Lösung PLM (enthaltend 10% - Pellitorin) | - | 0,020 |

Bei einer Dosierung von 1% der Aromakomposition in dieser Zahnpasta verbessert sich - im Vergleich zu dem nicht erfindungsgemäßen Aroma - das sensorische Profil deutlich und der Gesamteindruck überzeugt durch vermehrte Frische.

### Beispiel 2.17: Mundwasserkonzentrat mit Aroma Wintergrüntyp

| **Bestandteil** | **Anteil [%]** |
|---|---|
| Ethylalkohol 96% | 42,00 |
| Cremophor RH 455 | 5,00 |
| Entionisiertes Wasser | 48,67 |
| Allantoin | 0,20 |
| Natriumsaccharin 450 | 0,10 |
| Colour L-Blue 5000 (1% in Wasser) | 0,03 |
| **Aroma Wintergrüntyp (Beispiel 2.4)** | **4,00** |

Bei einer Dosierung von 4% der Aromakomposition in diesem Mundwasser verbessert sich - im Vergleich zu dem nicht erfindungsgemäßen Aroma - der Geschmack überzeugend durch eine stärkere und schnellere Kühlung im Mundraum.

### Beispiel 2.18: Mundwasser ('ready to use' ohne Alkohol) mit Aroma Eucalyptustyp

| **Bestandteil** | **I (Gew.-%)** | **II (Gew.-%)** |
|---|---|---|
| Cremophor RH 455 | 1,80 | 1,80 |
| Entionisiertes Wasser | 87,57 | Ad 100 |
| Sorbitol 70% | 10,00 | 10,00 |
| Natriumfluorid | 0,18 | 0,18 |
| Natriumsaccharin 450 | 0,10 | 0,10 |
| Solbrol M Natriumsalz | 0,15 | 0,15 |
| **Aroma Eucalyptustyp (Beispiel 2.6)** | **0,2** | **0,2** |
| Pellitorin-Lösung PLM (enthaltend 10% Pellitorin) | - | 0,0125 |

### Beispiel 2.19: Mundwasser ('ready to use' mit Alkohol)

| **Bestandteil** | **I (Gew.-%)** | **II (Gew.-%)** | **III (Gew.-%)** |
|---|---|---|---|
| Ethylalkohol 96% | 10,00 | 5,00 | 7,00 |
| Cremophor CO 40 | 1,00 | 1,00 | 1,00 |
| Benzoesäure | 0,10 | 0,12 | 0,10 |
| Entionisiertes Wasser | 83,46 | Ad 100 | Ad 100 |
| Sorbitol 70% | 5,00 | 1,00 | 5,00 |
| Natriumsaccharin 450 | 0,07 | 0,05 | 0,05 |
| L-Blue 5000 (1% in Wasser) | 0,10 | 0,10 | 0,10 |
| Glycerin | - | 8,00 | - |
| 1,2-Propylenglycol | - | 2,00 | 3,00 |
| Cetylpyridiniumchlorid | - | - | 0,07 |
| Wasserstoffperoxid (35% H₂O₂ in Wasser) | - | 3,00 | 4,00 |
| **Aroma Wintergrüntyp Cool (Beispiel 2.5)** | **0,25** | **-** | **-** |
| **Aroma Eucalyptustyp Cool (Beispiel 2.7)** | **-** | **0,25** | **0,05** |
| **Aroma Typ Eisbonbon II (Beispiel 2.10)** | **-** | **-** | **0,30** |

### Beispiel 2.20: Bonbon ('Hardboiled candy'), zuckerfrei

| **Bestandteil** | **I (Gew.-%)** | **II (Gew.-%)** | **II (Gew.-%)** |
|---|---|---|---|
| Wasser | 2,24 | 2,24 | 2,24 |
| Isomalt | 94,98 | Ad 100 | Ad 100 |
| Xylitol | 2,40 | 2,40 | 2,40 |
| Sucralose | 0,03 | 0,03 | 0,03 |
| Acesulfam K | 0,050 | 0,050 | 0,050 |
| Citronensäure | 0,050 | 0,050 | 0,050 |
| Pellitorin-Lösung PLM (enthaltend 10% Pellitorin) | - | 0,0075 | 0,010 |
| **Aroma Typ Eisbonbon II (Beispiel 2.10)** | **-** | **-** | **0,20** |
| **Aroma Pfefferminztyp (Beispiel 2.1)** | **0,25** | **0,20** | **-** |

### Beispiel 2.21: Bonbons ('Hardbolled candy')

| **Bestandteil** | **I (Gew.-%)** | **II (Gew.-%)** | **III (Gew.-%)** |
|---|---|---|---|
| Wasser | 2,75 | 2,50 | 2,50 |
| Zucker | 60,1 | Ad 100 | Ad 100 |
| Glucosesirup | 36,9 | 36,0 | 36,0 |
| Maltose | - | 2,00 | 2,00 |
| Palmkernöl | - | 0,80 | 0,80 |
| Citronensäure | - | 0,25 | 0,25 |
| Ginseng Extrakt | - | 0,40 | 0,40 |
| Blauer Farbstoff | - | 0,01 | 0,01 |
| **Aroma Krauseminztyp (Beispiel 2.3)** | **0,25** | **0,35** | - |
| **Aroma Typ Eisbonbon I (Beispiel 2.10)** | **-** | **-** | **0,175** |

### Beispiel 2.22: Zahncreme und Mundwasser als 2-in-1 Produkt

| | **I (Gew.-%)** | **II (Gew.-%)** |
|---|---|---|
| Ethanol, 96%ig | 5,00 | 5,00 |
| Sorbitol, 70 %ig in Wasser | 40,00 | 40,00 |
| Glycerin | 20,00 | 20,00 |
| Saccharin | 0,20 | 0,20 |
| Na-Monofluorphosphat | 0,76 | 0,76 |
| Solbrol M, Na-Salz | 0,15 | 0,15 |
| Abrasivkieselsäure (Sident 9) | 20,00 | 20,00 |
| Verdickungskieselsäure (Sident 22S) | 2,00 | 2,00 |
| Na-Carboxymethylcellulose | 0,30 | 0,30 |
| Natriumlaurylsulfat | 1,20 | 1,20 |
| Grüner Farbstoff (1%ig in Wasser) | 0,50 | 0,50 |
| **Aroma Eucalyptustyp Cool (Beispiel 2.7)** | **1,00** | **-** |
| Natürliches Pfefferminzöl Arvensis (enthält 68 Gew.-% an I-Menthol) | - | 1,12 |
| **2,6-Diethyl-5-sec-butyl-2-methyltetrahydropyran** | **-** | **0,08** |
| Wasser dest. | Ad 100 | Ad 100 |

### Beispiel 2.23: Instant-Getränkepulver

| | **I (Gew.-%)** | **II (Gew.-%)** |
|---|---|---|
| Zucker (Saccharose) | Ad 100 | Ad 100 |
| Citronensäure | 11,58 | 11,58 |
| Trinatriumcitrat | 0,70 | 0,70 |
| Tricalciumphosphat | 0,60 | 0,60 |
| Vitamin C | 0,66 | 0,66 |
| Grindsted^{®} JU 543 Stabilizer System (Danisco) | 0,90 | 0,90 |
| Saccharin | 0,561 | 0,561 |
| Citronenaroma, sprühgetrocknet | 1,75 | - |
| Orangenaroma, sprühgetrocknet | - | 1,85 |
| **Aroma Pfefferminztyp Cool (Beispiel 2.2),** sprüh- getrocknet auf Maltodextrin (DE 18), Dextrose und Gummi Arabicum, Aromabeladung 35% | **1,75** | **-** |
| **Aroma Zimttyp Cool (Beispiel 2.9),** sprühgetrock- net auf Maltodextrin (DE 15-19) und Gummi Arabicum, Aromabeladung 40% | **-** | **1,20** |

45 g dieses Instant-Getränkepulvers wurden in 1000 mL unter Rühren gelöst. Das erhaltene Getränk hatte einen erfrischenden, kühlenden Geschmack von Citrone und Pfefferminze (Variante I) bzw. einen erfrischenden, kühlenden Geschmack von Orange, Zimt und Minze (Variante II).

### Beispiel 2.24: Halsbonbons mit flüssig-viskoser Kernfüllung (center-filled hard candy) mit Aroma Zimttyp und Aroma Zimttyp Cool

| | **I (Gew.-%)** | **II (Gew.-%)** |
|---|---|---|
| **Mischung A (Hülle)** (80% der Bonbons) | | |
| Zucker (Saccharose) | 58,12 | 49,37 |
| Glucosesirup (Feststoffgehalt 80%) | 41,51 | 49,37 |
| **Aroma Zimttyp (Beispiel 2.8)** | **0,17** | **0,25** |
| I-Menthol | 0,10 | - |
| Citronenöl | 0,10 | 0,10 |
| Citronensäure | - | 0,91 |
| Total: | 100 | 100 |
| **Mischung B (Kern)** (20% der Bonbons) | | |
| High Fructose Maissirup (Gehalt an festen Zuckern 85%, knapp 15% Wasser) | 84,355 | 84,31 |
| Glycerin | 15,0 | 15,0 |
| Lecithin | 0,02 | 0,02 |
| Zimtöl | - | 0,27 |
| **Aroma Zimttyp Cool (Beispiel 2.9)** | **0,28** | - |
| **2,6-Diethyl-5-sec.-butyl-2-methyltetrahydropyran** | - | **0,05** |
| Capsaicin | 0,025 | - |
| Piperin | 0,05 | 0,05 |
| Vanillylalkohol-n-butylether | - | 0,10 |
| Roter Farbstoff, als 2,5%ige wässrige Lösung | 0,20 | 0,20 |
| Vanillin | 0,07 | - |
| Total | 100 | 100 |

In Anlehnung an die in US 6,432,441 (dort Beispiel 1) sowie die in US 5,458,894 bzw. US 5,002,791 beschriebenen Verfahren wurden Bonbons mit flüssig-viskosem Kern hergestellt. Beide Mischungen A und B wurden separat zu Basen für Hülle (Mischung A) bzw. Kern (Mischung B) verarbeitet. Die mittels Co-Extrusion erhaltenen gefüllten Halsbonbons wirkten bei betroffenen Personen beim Verzehr gegen Husten, Halsschmerzen und Heiserkeit.

### Beispiel 2.25: Zum Direktverzehr geeignete Gelatinekapseln

| | **I (Gew.-%)** | **II (Gew.-%)** | **III (Gew.-%)** |
|---|---|---|---|
| Gelatinehülle: | | | |
| Glycerin | 2,014 | 2,014 | 2,014 |
| Gelatine 240 Bloom | 7,91 | 7,91 | 7,91 |
| Aspartam | 0,05 | - | - |
| Sucralose | 0,035 | 0,050 | 0,070 |
| Allura Red (roter Farbstoff) | 0,006 | 0,006 | 0,006 |
| Brillant Blue (blauer Farbstoff) | 0,005 | 0,005 | 0,005 |
| | | | |
| Kernzusammensetzung: | | | |
| Pflanzenöltriglyceride (Kokonussölfraktion; coconut oil fraction) | Ad 100 | Ad 100 | Ad 100 |
| Aroma G | 9,95 | - | 7,5 |
| **Aroma Pfefferminztyp Cool (Beispiel 2.2),** | **-** | **15,0** | **18,5** |
| **2,6-Diethyl-5-isopropyl-2-methyl-tetrahydropyran** | **0,60** | **-** | **-** |

Aroma G hatte dabei folgende Zusammensetzung (Angaben jeweils in Gew.-%): 0, 1 % Neotam Pulver, 29,3% Pfefferminzöl arvensis, Ad 100% Pfefferminz piperita Öl Willamette, 2,27% Sucralose, 0,7% Gewürznelkenöl (clove bud oil), 2,28% Triacetin, 5,4% Diethyltartrat, 12,1% Pfefferminzöl yakima, 0,7% Ethanol, 3,36% 2-Hydroxyethylmenthylcarbonat, 2,6% 2-Hydroxypropylmenthylcarbonat, 5,77% D-Limonen, 5,67% L-Menthylacetat, 0,4% Vitamin E - acetat.

Die zum Direktverzehr geeigneten Gelatinekapseln I, II, III wurden gemäß WO 2004/050069 hergestellt und hatten jeweils einen Durchmesser von 5 mm, das Gewichtsverhältnis von Kernmaterial zu Hüllmaterial lag bei 90 : 10. Die Kapseln öffneten sich jeweils im Mund innerhalb von weniger als 10 Sekunden und lösten sich vollständig innerhalb von weniger als 50 Sekunden auf.

### Beispiel 2.26: Kaugummis (mit Zucker und zuckerfrei)

| | **I (Gew.-%)** | **II (Gew.-%)** | **III (Gew.-%)** |
|---|---|---|---|
| Kaugummibase | 21,0 | 30,0 | 30,0 |
| Glycerin | 0,5 | 1,0 | 1,0 |
| Menthol-Spearmint-Eucalyptus Aroma P1 | 1,0 | 1,4 | 1,4 |
| Glucosesirup | 16,5 | - | - |
| Puderzucker | Ad 100 | - | - |
| **2,6-Diethyl-5-isopropyl-2-methyl-tetrahydropyran** | | **-** | **0,15** |
| **2,6-Dimethyl-5-isopropyl-2-thyltetrahydropyran** | **0,10** | **-** | **-** |
| **2,6-Diethyl-5-sec.-butyl-2-methyltetrahydropyran** | **-** | **0,125** | **-** |
| Sorbitol (in Pulverform) | - | Ad 100 | Ad 100 |
| Palatinit | - | 9,5 | 9,5 |
| Xylitol | - | 2,0 | 2,0 |
| Mannitol | - | 3,0 | 3,0 |
| Aspartam | - | 0,1 | 0,1 |
| Acesulfam K | - | 0,1 | 0,1 |
| Emulgum™ (Emulgator) | - | 0,3 | 0,3 |
| Sorbitol 70%, in Wasser | - | 14,0 | 14,0 |

### Aroma P1 hatte folgende Zusammensetzung (Angaben jeweils in Gew.-%):

0,05% Isobutyraldehyd, 0,05% 3-Octanol, 0,05% Dimethylsulfid, 0,1% trans-2-Hexenal, 0,1% cis-3-Hexenol, 0, 1 % natürliches 4-Terpineol, 0, 1 % Isopulegol, 0,2% natürliches Piperiton, 0,3% Linalool, 1,0% Isoamylalkohol, 1,0% Isovaleraldehyd, 2,5% natürliches alpha-Pinen, 2,5% natürliches beta-Pinen, 8,0% Eucalyptol, 7,0% I-Menthylacetat, 12,0% I-Menthon, 5,0% Isomenthon, 20,5% I-Carvon, 39,45% I-Menthol.

### Beispiel 2.27: Zuckerfreie Kaugummis

Die Kaugummibase K1 bestand aus 2,0% Butyl Rubber (Isobuten-Isopren-Copolymer, MW 400,00),400000), 6,0% Polyisobuten (MW = 43,800), 43,5% Polyvinylacetat (MW = 12,000), 31,5% Polyvinylacetat (MW = 47,000), 6,75% Triacetin und 10,25% Calciumcarbonat. Die Herstellung der Kaugummibase K1 und der Kaugummis kann analog zu US 5,601,858 erfolgen.

| | **I (Gew.-%)** | **II (Gew.-%)** | **III (Gew.-%)** |
|---|---|---|---|
| Kaugummibase K1 | 26,00 | 27,00 | 26,00 |
| Triacetin | 0,25 | 0,25 | 0,25 |
| Lecithin | 0,50 | 0,50 | 0,50 |
| Sorbitol, kristallin | Ad 100 | Ad 100 | Ad 100 |
| Mannitol | 15,30 | 15,20 | 15,10 |
| Glycerin | 12,10 | 12,00 | 11,80 |
| Saccharin-Na | 0,17 | - | 0,10 |
| Verkapseltes Aspartam | 1,08 | 1,18 | 1,08 |
| Amorphes Silica | 1,00 | 1,00 | 1,00 |
| Baumwollsamenöl | 0,50 | 0,50 | 0,50 |
| Polyoxyethylen-sorbitan-monolaurat (E-432) | 1,00 | 1,00 | 1,00 |
| Verkapseltes I-Carvon (Beladung: 30%) | - | 0,20 | - |
| **Aroma Wintergrüntyp Cool (Beispiel 2.5),** | **1,00** | **-** | **1,70** |
| **Aroma Eucalyptustyp Cool (Beispiel 2.7)** | **0,50** | **1,40** | **-** |
| **2,6-Dimethyl-5-isopropyl-2-ethyltetrahydropyran** | - | **0,10** | **-** |
| I-M enthyl-I-lactat | - | - | 0,20 |

### Beispiel 2.28: Zuckerfreie Kaugummis

Die Kaugummibase K2 bestand aus 28,5% Terpenharz, 33,9% Polyvinylacetat (MW = 14,000), 16,25% hydriertes Pflanzenöl, 5,5% Mono- und Diglyceride, 0,5% Polyisobuten (MW 75,000), 2,0% Butyl Rubber (Isobuten-Isopren-Copolymer), 4,6% amorphes Siliciumdioxid (Wassergehalt ca. 2,5%), 0,05% Antioxidans tert.-Butylhydroxytoluol (BHT), 0,2% Lecitihin, und 8,5% Calciumcarbonat. Die Herstellung der Kaugummibase K2 und der Kaugummis kann analog zu US 6,986,907 erfolgen.

| | **I** (Gew.-%) | **II** (Gew.-%) | **III** (Gew.-%) |
|---|---|---|---|
| Kaugummibase K2 | 25,30 | 27,30 | 26,30 |
| Sorbitol | Ad 100 | Ad 100 | Ad 100 |
| Glycerin | 2,40 | 2,40 | 2,40 |
| Lecithin | 7,00 | 7,00 | 7,00 |
| Aspartam | 0,14 | 0,14 | 0,14 |
| Verkapseltes Aspartam | 0,68 | 0,68 | 0,68 |
| Menthol, sprühgetrocknet (Beladung: 25%) | 0,50 | - | 0,50 |
| Kirscharoma, sprühgetrocknet (enthält Benzaldehyd) | - | 1,00 | - |
| **Aroma Pfefferminztyp (Beispiel 2.1),** sprühgetrocknet, Aromagehalt 30% | **1,50** | **1,70** | **-** |
| **Aroma Zimttyp Cool (Beispiel 2.9)** | **1,00** | **-** | **1,50** |

Die Kaugummis der Rezeptur (I) und (II) wurden als Streifen, die der Rezeptur (III) als Kompaktate in Kissenform hergestellt und anschließend mit Xylit dragiert.

### Beispiel 2.29: Zahncremes mit Bleichwirkung

| | **I (Gew.-%)** | **II (Gew.-%)** | **III (Gew.-%)** |
|---|---|---|---|
| Polyphosphat (Glass H, (n = 21), Astaris) | 7,00 | 7,00 | 7,00 |
| Calciumperoxid | 1,00 | - | 2,50 |
| Na-percarbonat | - | 11,00 | - |
| Poloxamer 407 | 5,00 | 2,00 | 5,00 |
| Polyethylenglycol | 3,00 | - | 3,00 |
| Sorbitol, 70 %ig in Wasser | - | 22,00 | - |
| Glycerin | 43,80 | 12,50 | 28,60 |
| 1,2-Propylenglycol | 4,00 | - | 2,50 |
| Na-Saccharin | 0,40 | 0,20 | 0,50 |
| Natrium bicarbonat | - | 5,00 | 15,00 |
| Natriumcarbonat | 2,00 | 2,00 | 2,00 |
| Silica | 20,00 | 22,00 | 20,00 |
| Na-Carboxymethylcellulose | 0,60 | 0,55 | 0,30 |
| Natriumlaurylsulfat | 1,00 | 4,00 | 2,00 |
| Xanthan Gum | 0,20 | 0,20 | 0,20 |
| Titandioxid (Anatas) | 0,50 | 0,50 | 0,50 |
| **Aroma Eucalyptustyp Cool (Beispiel 2.7)** | **1,00** | **-** | **-** |
| **Aroma Zimttyp Cool (Beispiel 2.9)** | **-** | **1,25** | **-** |
| **Aroma Pfefferminztyp Cool (Beispiel 2.2)** | **-** | **-** | **1,50** |
| Wasser dest. | Ad 100 | Ad 100 | Ad 100 |

### Beispiel 2.30: Zahncremes mit Zinn- und Zinksalzen

| | **I (Gew.-%)** | **II (Gew.-%)** | **III (Gew.-%)** |
|---|---|---|---|
| Natriumfluorid NaF | 0,42 | 0,50 | - |
| Zinnfluorid SnF₂ | - | 0,90 | 0,95 |
| Zinnchlorid SnCl₂ | 1,50 | - | 2,00 |
| Zinklactat | 2,00 | 2,00 | - |
| Zinkcarbonat ZnCO₃ | - | 1,00 | 1,50 |
| Na-gluconat | - | 0,67 | 1,50 |
| Poloxamer 407 | 14,50 | - | - |
| Polyethylenglycol | 1,00 | 3,00 | - |
| Sorbitol, 70 %ig in Wasser | - | 38,00 | 37,50 |
| Glycerin | 37,50 | 5,00 | 14,40 |
| 1,2-Propylenglycol | 7,00 | 5,00 | - |
| Na-Saccharin | 0,30 | 0,50 | 0,50 |
| Abrasiv-Silica | 20,00 | 22,50 | 25,00 |
| Natriumhydroxid | - | 0,10 | 0,20 |
| Natriumlaurylsulfat | - | 2,00 | 1,50 |
| Na-polyphosphat | - | - | 4,00 |
| Tetranatriumpyrophosphat | 1,00 | 2,50 | - |
| Farbstoff (1%ig in Wasser) | 0,40 | 0,50 | 0,50 |
| **Aroma Eucalyptustyp Cool (Beispiel 2.7)** | **0,95** | **-** | **-** |
| **Aroma Zimttyp Cool (Beispiel 2.9)** | **-** | **1,20** | **-** |
| **Aroma Pfefferminztyp Cool (Beispiel 2.2)** | **-** | **-** | **1,15** |
| Wasser dest. | Ad 100 | Ad 100 | Ad 100 |

## Patentansprüche

1. Alkyliertes Tetrahydropyran der Formel (I): 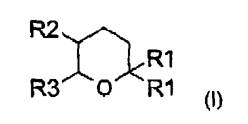 wobei jedes R1 unabhängig voneinander entweder Wasserstoff oder ein verzweigter oder unverzweigter Alkylrest mit 1 bis 3 C-Atomen oder ein verzweigter oder unverzweigter Alkenylrest mit 2 bis 3 C-Atomen,
R2 ein verzweigter oder unverzweigter Alkyl oder Alkenylrest mit 3 bis 4 C-Atomen, und R3 ein verzweigter oder unverzweigter Alkylrest mit 1 bis 5 C-Atomen ist,
ausgenommen 2,6.Dimethyl-3-(1-methylethenyl)tetrahydropyran, 6-Methyl-3-(1-methylethenyl)-2-(2-methylpropyl)-tetrahydropyran. 2-Msthyl-3-n-propyl-tetrahydropyran und 2-Methyl-3-n-butyl-tetrahydropyran.

2. Alkyliertes Tetrahydropyran nach Anspruch 1, wobei
jedes R1 unabhängig voneinander ein Methyl-, Ethyl- oder Vinylrest ist,
R2 ein Isopropyl-, Isopropenyl-, sec-Butyl- oder sec-Butenylrest ist, und
R3 ein unverzweigter Alkylrest mit 1 bis 3 C-Atomen Ist.

3. Alkyliertes Tetrahydropyran nach einem der vorherigen Ansprüche, wobei jedes R1 unabhängig voneinander ein Methyl- oder Ethylrest ist,
R2 ein Isopropyl- oder sec-Butytrest ist, und
R3 ein Methyl- oder Ethylrest ist.

4. Alkyliertes Tetrahydropyran nach einem der vorherigen Ansprüche, ausgewählt aus der Gruppe bestehend aus 2.6-Diethyl-5-Isopropyl-2-methyltetrahydropyran, 6-Ethyl-5-isopropenyl-2-methyl-2-vinyl tetrahydropyran, 2,6-Dimethyl-5-isopropyl-2-ethyltetrahydropyran. 2,6-Dimethyl-5-isopropenyl-2-vinyltetrahydropyran, 2,6-Diethyl-5-sec.-butyl-2-methyltetrahydropyran und 2,6-Dimethyl-5-sec.-butyl-2-ethyltetrahydropyran.

5. Mischung umfassend oder bestehend aus
a) einem, zwei, drei, vier oder mehr alkylierten retrahydropyranen gemäß einem der Ansprüche 1 bis 4,
b) einem oder mehreren (flüchtigen) Aroma- und/oder Geschmacksstoffen, insbesondere einer oder mehreren Substanz(en) ausgewählt aus der Gruppe bestehend aus Substanzen mit physiologischer Kühlwirkung, Aromastoffe ohne physiologische Kühlwirkung, trigeminal oder mundwässernd wirksame Substanzen ohne physiologische Kühlwirkung, und geschmacksmodulierende Substanzen,
c) und optional einem kosmetisch oder pharmazeutisch akzeptablen Träger.

6. Mischung nach Anspruch 5, wobei
- eine oder mehrere der Substanzen mit physiologischer Kühlwirkung ausgewählt sind aus der Gruppe bestehend aus 1-Menthol, Isopulegol, Menthonglycerinacetal, Menthyllactat, substituierten Menthan-3-carbonsäureamiden, substituierten N-Aryl-menthan-3-carbonsäureamiden, substituierten N-Alkyl-menthan-3-carbonsäureamiden, 2-Isopropyl-N,2,3-trimethylbutanamid, substituierten Cyclohexancarbonsäureamiden, 3-Menthoxy-1,2-propandiol, 2-Hydroxyethylmenthylcarbonat, 2-Hydroxypropylmenthylcarbonat, N-Acetylglycinmenthylester, Menthythydroxycarbonsäureestern, Menthylmonosuccinat, Menthylmonoglutarat, 2-Mercaptocyclodecanon, Menthyl-2-pyrrolidin-5-oncarboxylat, [N-(4-Cyanomethylphenyl)-p-menthanecarboxamid], (1 R,2S,5R)-2-isopropyl-5-methyl-N-(2-(pyridin-2-yl)ethyl)cyclohexanecarboxamid, WS-3, WS-23, WS-5, WS-12, WS-14, TPG1^{™}, Frescomenthe, p-Menthane-3,8-diol, N,N-Dimethyl menthyl succinamid, 6-isopropyl-3,9-dimethyl-1,4-dioxaspiro[4.5]decan-2-on, Icilin und Icilinderivate, Cubebol, Menthyloxamat, Menthyl-N-methyloxamat, Menthyl-N,N-dimethyloxamat, Menthyl-N-ethyloxamat, Menthyl-N,N-diethyloxamat, Menthyl-N-propyloxamat, Menthyl-N,N-dipropyloxamat. Menthyl-N-isopropyloxamat, Menthyl-N,N-diisopropyloxamat, Menthyl-N-cyclopropyloxamat, Menthyl-N-butyloxamat, Morpholin-4-yl-oxo-essigsäure-(1R,2S,5R)-2-isopropyl-5-methyl-cyclohexylester. Menthyl-N-(2-methoxyethyl)-oxamat, Menthyl-N-(3-methoxypropyl)-oxamat, Menthyl-N-(2-hydroxyethyl)-oxamat, Menthyl-N-(3-hydroxypropyl)-oxamat.
- einer oder mehrere der Aromastoffe ohne physiologische Kühlwirkung ausgewählt sind aus der Gruppe bestehend aus einen scharfen Geschmack oder eine Wärme- oder Hitzempfindung auf Haut und Schleimhäuten oder ein Prickel- oder Kribbelgefühl im Mund- und Rachenraum hervorrufenden Substanzen.

7. Mischung nach einem der Ansprüche 5 oder 6, wobei einer oder mehrere der Substanzen im Bestandteil b) (i) einen geschmacklichen Effekt verursachen oder (ii) keinen geschmacklichen Effekt verursachen.

8. Der Ernährung, der Mundhygiene oder dem Genuss dienende Zubereitung oder pharmazeutische oder kosmetische Zubereitung, umfassend (i) ein oder mehrere alkylierte Tetrahydropyrane gemäß einem der Ansprüche 1 bis 4 oder (ii) eine Mischung nach einem der Ansprüche 5 bis 7, wobei der Bestandteil (i) oder, wenn vorhanden, (ii) vorliegt in einer ausreichenden Konzentration
- zum Erreichen einer physiologischen Kühlwirkung auf der Haut und/oder einer Schleimhaut und/oder
- zum Vermitteln, Modifizieren oder Verstärken eines Frischegefühls in Mund, Rachen und/oder den Atemwegen.

9. Zubereitung nach Anspruch 8, wobei die Zubereitung
(i) eine der Ernährung oder dem Genuss dienende Zubereitung ist ausgewählt aus Backwaren, Süßwaren, alkoholische oder nicht-alkoholische Getränke, Instantgetränke, Fleischprodukte, Eier oder Eiprodukte , Getreideprodukte, Milchprodukte, Fruchtzubereitungen, Gemüsezubereitungen, Knabberartikel, Produkte auf Fett- und Ölbasis oder Emulsionen derselben, sonstige Fertiggerichte und Suppen, Gewürze, Würzmischungen, Aufstreuwürzungen, Halbfertigwaren, Nahrungsergänzungsmittel; oder
(ii) eine der Mundhygiene dienende Zubereitung ist, vorzugsweise auf Basis eines Zahnpflegemittels, und ausgewählt ist aus der Gruppe bestehend aus: Zahnpasta. Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide und Zahnpflegekaugummi; oder
(iii) eine pharmazeutische Zubereitung ist, wobei die Zubereitung eine vorzugsweise orale oder nasale pharmazeutische Zubereitung ist, vorzugsweise in Form von Kapseln, Tabletten, Dragees. Granulaten, Pellets, Feststoffmischungen, Dispersionen in flüssigen Phasen, als Emulsionen, als Pulver, als Lösungen, als Pasten oder als andere schluck- oder kaubare Zubereitung: oder
(iv) eine kosmetische Zubereitung ist, ausgewählt aus der Gruppe bestehend aus: Seife, Syndet, flüssiges Wasch-, Dusch-, oder Badepräparat, Emulsion, Salbe, Paste, Gel, Öl, Toner, Balsam, Serum, Puder, Eau de Toilette, Toilette, Eau de Cologne, Parfum, Wachs, Stift, Roll-On, (Pump-)Spray, Aerosol (schäumend, nicht schäumend oder nachschäumend), Fußpflegemittel, Bartreinigungs- oder -pflegemittel, Insekten abwehrendes Mittel, Sonnenschutzmittel, Aftersun-Präparat, Rasiermittel, After-Shave, Haarentfernungsmittel, Haarpflegemittel, Conditioner, Haarkur, Haarwasser, Haarspülung, Frisiercreme, Pomade, Dauerwell- und Fixierungsmittel, Haarglättungsmittel, Haarfestiger, Styling-Hilfe, Blondiermittel, Haaraufheller, Haarconditioner, Haarmousse, Haartönung, Nagelpflegemittel, Deodorant, Antitranspirant, Mundwasser, Munddusche, Make-Up, Make-Up-Entferner, Augenpflege, Lippenkosmetika, Lippenpflegemittel, dekorative Kosmetik, Badeartikel und Maske.

10. Verwendung (i) eines alkylierten Tetrahydropyrans der Formel (I): 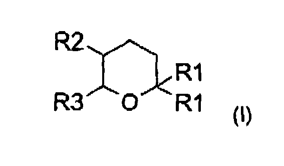 wobei jedes R1 unabhängig voneinander entweder Wasserstoff oder ein verzweigter oder unverzweigter Alkylrest mit 1 bis 3 C-Atomen oder ein verzweigter oder unverzweigter Alkenylrest mit 2 bis 3 C-Atomen,
und R2 ein verzweigter oder unverzweigter Alkyl oder Alkenylrest mit 3 bis 4 C-Atomen und R3 ein verzweigter oder unverzweigter Alkylrest mit 1 bis 5 C-Atomen ist,
oder (ii) einer Mischung nach einem der Ansprüche 5 bis 7
- zum Erzeugen einer Kühlwirkung auf der Haut und/oder einer Schleimhaut zu anderen als therapeutischen Zwecken, und/oder
- zum Vermitteln, Modifizieren oder Verstärken eines Frischegefühls in Mund, Rachen und/oder den Atemwegen zu anderen als therapeutischen Zwecken.

11. Verwendung (i) eines alkylierten Tetrahydropyrans der Formel (I): 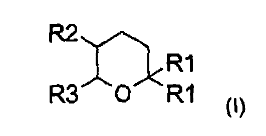 wobei jedes R1 unabhängig voneinander entweder Wasserstoff oder ein verzweigter oder unverzweigter Alkylrest mit 1 bis 3 C-Atomen oder ein verzweigter oder unverzweigter Alkenylrest mit 2 bis 3 C-Atomen.
und R2 ein verzweigter oder unverzweigter Alkyl oder Alkenylrest mit 3 bis 4 C-Atomen und R3 ein verzweigter oder unverzweigter Alkylrest mit 1 bis 5 C-Atomen ist,
oder (ii) einer Mischung nach einem der Ansprüche 5 bis 7
zum Herstellen eines Arzneimittels.

12. Verwendung nach Anspruch 11 zum Herstellen eines Arzneimittels, wobei das herzustellende Arzneimittel der Bekämpfung oder Linderung von Husten-, Schnupfen-, Entzündungs-, Halsschmerz- oder Heiserkeitssymptomen dient.

13. Nicht-therapeutisches Verfahren (a) zum Erzeugen einer Kühlwirkung auf der Haut und/oder einer Schleimhaut und/oder (b) zum Vermitteln, Modifizieren oder Verstärken eines Frischegefühls in Mund, Rachen und/oder den Atemwegen, mit folgendem Schritt: Applizieren einer zum Erreichen der Wirkung (a) und/oder (b) ausreichenden Menge (i) eines alkylierten Tetrahydropyrans der Formel (I): 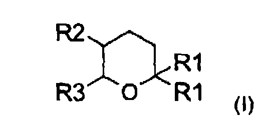 wobei jedes R1 unabhängig voneinander entweder Wasserstoff oder ein verzweigter oder unverzweigter Alkylrest mit 1 bis 3 C-Atomen oder ein verzweigter oder unverzweigter Alkenylrest mit 2 bis 3 C-Atomen,
und R2 ein verzweigter oder unverzweigter Alkyl oder Alkenylrest mit 3 bis 4 C-Atomen und R3 ein verzweigter oder unverzweigter Alkylrest mit 1 bis 5 C-Atomen ist,
oder (ii) einer Mischung nach einem der Ansprüche 5 bis 7 auf die Haut und/oder eine Schleimhaut.

14. Verfahren zum Herstellen eines alkylierten Tetrahydropyrans der Formel (I): 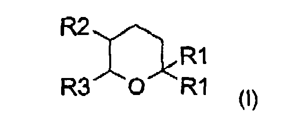 wobei jedes R1 unabhängig voneinander entweder Wasserstoff oder ein verzweigter oder unverzweigter Alkylrest mit 1 bis 3 C-Atomen oder ein verzweigter oder unverzweigter Alkenylrest mit 2 bis 3 C-Atomen,
und R2 ein verzweigter oder unverzweigter Alkyl oder Alkenylrest mit 3 bis 4 C-Atomen und R3 ein verzweigter oder unverzweigter Alkylrest mit 1 bis 5 C-Atomen ist,
umfassend die Schritte:
- Umsetzen des Aldehyds (B) mit der Alkoholkomponente (A) zu dem entsprechenden cyclisierten Tetrahydropyranverbindung (C),
- gegebenenfalls Reduzieren der Tetrahydropyranverbindung (C) zu der Tetrahydropyranverbindung (D) 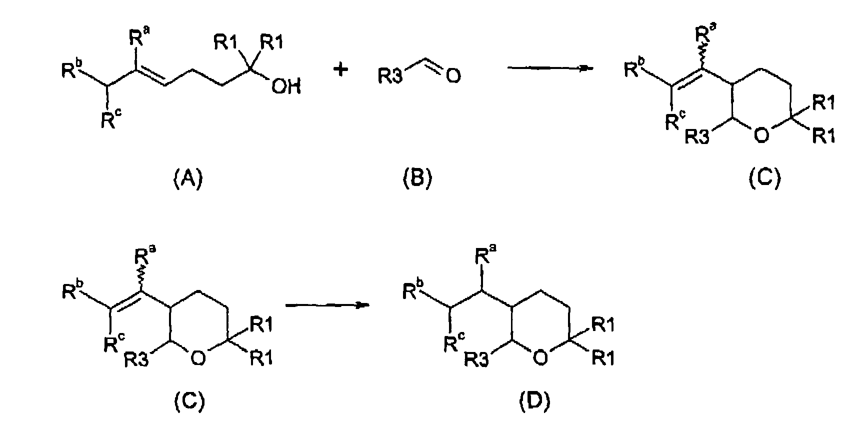
wobei
R1 und R3 jeweils die oben genannte (gegebenenfalls bevorzugte) Bedeutung haben, und
R^{a}, R^{b} und R^{c} unabhängig voneinander Wasserstoff, Methyl oder Ethyl darstellen können, mit der Maßgabe, dass R^{a}, R^{b} und R^{c} in Summe 1 oder 2 C-Atome enthalten.

## Claims

1. Alkylated tetrahydropyran of the formula (I): 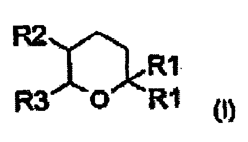 wherein each R1 independently of each other is either hydrogen or a branched or unbranched alkyl radical having 1 to 3 C atoms or a branched or unbranched alkenyl radical having 2 to 3 C atoms,
R2 is a branched or unbranched alkyl or alkenyl radical having 3 to 4 C atoms, and
R3 is a branched or unbranched alkyl radical having 1 to 5 C atoms,
excluding 2,6-dimethyl-3-(1-methylethenyl)-tetrahydropyran, 6-methyl-3-(1-methylethenyl)-2-(2-methylpropyl)-tetrahydropyran, 2-methyl-3-n-propyl-tetrahydropyran and 2-methyl-3-n-butyl-tetrahydropyran.

2. Alkylated tetrahydropyran according to claim 1, wherein
each R1 independently of each other is a methyl, ethyl or vinyl radical,
R2 is an isopropyl, isopropenyl, sec-butyl or sec-butenyl radical, and
R3 is an unbranched alkyl radical having 1 to 3 C atoms.

3. Alkylated tetrahydropyran according to one of the preceding claims, wherein
each R1 independently of each other is a methyl or ethyl radical,
R2 is an isopropyl or sec-butyl radical, and
R3 is a methyl or ethyl radical.

4. Alkylated tetrahydropyran according to one of the preceding claims, chosen from the group consisting of 2,6-diethyl-5-isopropyl-2-methyltetrahydropyran, 6-ethyl-5-isopropenyl-2-methyl-2-vinyltetrahydropyran, 2,6-dimethyl-5-isopropyl-2-ethyltetrahydropyran, 2,6-dimethyl-5-isopropenyl-2-vinyltetrahydropyran, 2,6-diethyl-5-sec-butyl-2-methyltetrahydropyran and 2,6-dimethyl-5-sec-butyl-2-ethyltetrahydropyran.

5. Mixture comprising or consisting of
a) one, two, three, four or more alkylated tetrahydropyrans according to one of claims 1 to 4,
b) one or more (volatile) aroma and/or flavour substances, in particular one or more substance(s) chosen from the group consisting of substances with a physiological cooling action, aroma substances without a physiological cooling action, substances without a physiological cooling action which have a trigeminal or mouth-watering action, and flavour-modulating substances,
c) and optionally a cosmetically or pharmaceutically acceptable carrier.

6. Mixture according to claim 5, wherein
- one or more of the substances with a physiological cooling action are chosen from the group consisting of 1-menthol, isopulegol, menthone glycerol acetal, menthyl lactate, substituted menthane-3-carboxylic acid amides, substituted N-aryl-menthane-3-carboxylic acid amides, substituted N-alkyl-menthane-3-carboxylic acid amides, 2-isopropyl-N,2,3-trimethylbutanamide, substituted cyclohexanecarboxylic acid amides, 3-menthoxy-1,2-propanediol, 2-hydroxyethyl menthyl carbonate, 2-hydroxypropyl menthyl carbonate, N-acetylglycine menthyl ester, menthyl hydroxycarboxylic acid esters, menthyl monosuccinate, menthyl monoglutarate, 2-mercaptocyclodecanone, menthyl 2-pyrrolidin-5-onecarboxylate, [N-(4-cyanomethylphenyl)-p-menthanecarboxamide], (1R,2S,5R)-2-isopropyl-5-methyl-N-(2-(pyridin-2-yl)ethyl)cyclohexanecarboxamide, WS-3, WS-23, WS-5, WS-12, WS-14, TPG1^{™}, frescomenthe, p-menthane-3,8-diol, N,N-dimethylmenthylsuccinamide, 6-isopropyl-3,9-dimethyl-1,4-dioxaspiro[4.5]decan-2-one, icilin and icilin derivatives, cubebol, menthyl oxamate, menthyl N-methyloxamate, menthyl N,N-dimethyloxamate, menthyl N-ethyloxamate, menthyl N,N-diethyloxamate, menthyl N-propyloxamate, menthyl N,N-dipropyloxamate, menthyl N-isopropyloxamate, menthyl N,N-diisopropyloxamate, menthyl N-cyclopropyloxamate, menthyl N-butyloxamate, morpholin-4-yl-oxo-acetic acid (1R,2S,5R)-2-isopropyl-5-methylcyclohexyl ester, menthyl N-(2-methoxyethyl)-oxamate, menthyl N-(3-methoxypropyl)-oxamate, menthyl N-(2-hydroxyethyl)-oxamate, menthyl N-(3-hydroxypropyl)-oxamate,
- one or more of the aroma substances without a physiological cooling action are chosen from the group consisting of substances which cause a sharp flavour or a sensation of warmth or heat on skin and mucous membranes or a prickling or tingling feeling in the oral and pharyngeal cavity.

7. Mixture according to one of claims 5 or 6, wherein one or more of the substances in constituent b) (i) cause a flavour effect or (ii) cause no flavour effect.

8. Formulation for nutrition, oral hygiene or consumption for pleasure or pharmaceutical or cosmetic formulation, comprising (i) one or more alkylated tetrahydropyrans according to one of claims 1 to 4 or (ii) a mixture according to one of claims 5 to 7, wherein constituent (i) or, if present, (ii) is present in a sufficient concentration
- to achieve a physiological cooling action on the skin and/or a mucous membrane and/or
- to impart, modify or intensify a feeling of freshness in the mouth, pharynx and/or the respiratory tract.

9. Formulation according to claim 8, wherein the formulation
(i) is a formulation for nutrition or consumption for pleasure chosen from baked goods, confectionery, alcoholic or non-alcoholic drinks, instant drinks, meat products, eggs or egg products, cereal products, dairy products, fruit formulations, vegetable formulations, nibbles, fat- and oil-based products or emulsions thereof, other ready-made dishes and soups, spices, spice mixtures, sprinkling spices, semi-finished goods, food supplements; or
(ii) is a formulation for oral hygiene, preferably based on a dental care composition, and is chosen from the group consisting of: toothpaste, dental cream, dental gel, dental powder, tooth-cleaning liquid, tooth-cleaning foam, mouthwash, dental cream and mouthwash as a 2-in-1 product, bonbon for sucking, oral spray, dental floss and dental care chewing gum; or
(iii) is a pharmaceutical formulation, the formulation being a preferably oral or nasal pharmaceutical formulation, preferably in the form of capsules, tablets, coated tablets, granules, pellets, solid mixtures, dispersions in liquid phases, emulsions, powders, solutions, pastes or another formulation which can be swallowed or chewed; or
(iv) is a cosmetic formulation chosen from the group consisting of: soap, syndet, liquid washing, showering or bathing preparation, emulsion, ointment, paste, gel, oil, toner, balsam, serum, powder, eau de toilette, toilette, eau de Cologne, perfume, wax, stick, roll-on, (pump) spray, aerosol (foaming, non-foaming or after-foaming), foot care composition, beard cleaning or care composition, insect-repellent composition, sunscreen composition, after-sun preparation, shaving composition, after-shave, depilatory composition, hair care composition, conditioner, hair tonic, hair lotion, hair rinse, styling cream, pomade, permanent wave and setting composition, hair smoothing composition, hair setting agent, styling aid, blonding composition, hair lightener, hair conditioner, hair mousse, hair tint, nail care composition, deodorant, antiperspirant, mouthwash, mouth spray, make-up, make-up remover, eye care, lip cosmetics, lip care composition, decorative cosmetics, bathing article and mask.

10. Use (i) of an alkylated tetrahydropyran of the formula (I) : 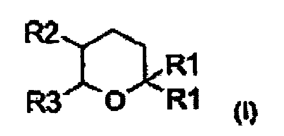 wherein each R1 independently of each other is either hydrogen or a branched or unbranched alkyl radical having 1 to 3 C atoms or a branched or unbranched alkenyl radical having 2 to 3 C atoms,
and R2 is a branched or unbranched alkyl or alkenyl radical having 3 to 4 C atoms
and R3 is a branched or unbranched alkyl radical having 1 to 5 C atoms,
or (ii) of a mixture according to one of claims 5 to 7
- for generating a cooling action on the skin and/or a mucous membrane for other than therapeutic purposes and/or
- for imparting, modifying or intensifying a feeling of freshness in the mouth, pharynx and/or the respiratory tract for other than therapeutic purposes.

11. Use (i) of an alkylated tetrahydropyran of the formula (I) : 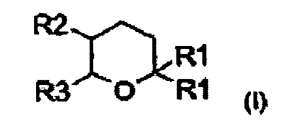 wherein each R1 independently of each other is either hydrogen or a branched or unbranched alkyl radical having 1 to 3 C atoms or a branched or unbranched alkenyl radical having 2 to 3 C atoms,
and R2 is a branched or unbranched alkyl or alkenyl radical having 3 to 4 C atoms
and R3 is a branched or unbranched alkyl radical having 1 to 5 C atoms,
or (ii) of a mixture according to one of claims 5 to 7
for the preparation of a medicament.

12. Use according to claim 11 for the preparation of a medicament, wherein the medicament to be prepared is for combating or alleviating cough, cold, inflammation, sore throat or hoarseness symptoms.

13. Non-therapeutic method (a) for generating a cooling action on the skin and/or a mucous membrane and/or (b) for imparting, modifying or intensifying a feeling of freshness in the mouth, pharynx and/or the respiratory tract, with the following step:
application of an amount sufficient to achieve the action (a) and/or (b) (i) of an alkylated tetrahydropyran of the formula (I): 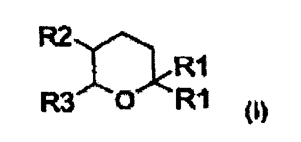 wherein each R1 independently of each other is either hydrogen or a branched or unbranched alkyl radical having 1 to 3 C atoms or a branched or unbranched alkenyl radical having 2 to 3 C atoms,
and R2 is a branched or unbranched alkyl or alkenyl radical having 3 to 4 C atoms
and R3 is a branched or unbranched alkyl radical having 1 to 5 C atoms,
or (ii) of a mixture according to one of claims 5 to 7, to the skin and/or a mucous membrane.

14. Process for the preparation of an alkylated tetrahydropyran of the formula (I): 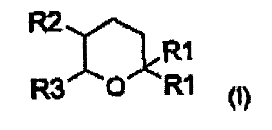 wherein each R1 independently of each other is either hydrogen or a branched or unbranched alkyl radical having 1 to 3 C atoms or a branched or unbranched alkenyl radical having 2 to 3 C atoms,
and R2 is a branched or unbranched alkyl or alkenyl radical having 3 to 4 C atoms
and R3 is a branched or unbranched alkyl radical having 1 to 5 C atoms,
comprising the steps:
- reaction of the aldehyde (B) with the alcohol component (A) to give the corresponding cyclized tetrahydropyran compound (C),
- optionally reduction of the tetrahydropyran compound (C) to give the tetrahydropyran compound (D) 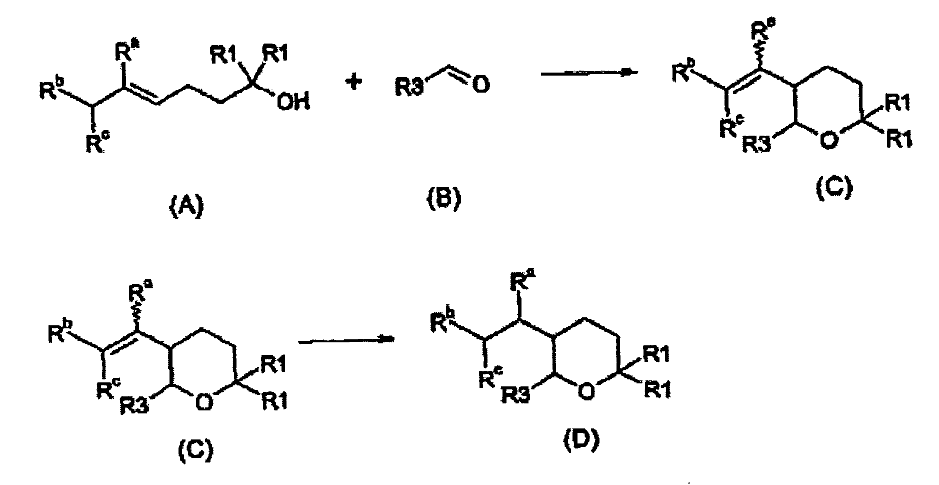
wherein
R1 and R3 in each case have the abovementioned (optionally preferred) meaning, and
R^{a}, R^{b} and R^{c} independently of each other can represent methyl or ethyl, with the proviso that R^{a}, R^{b} and R^{c} contain 1 or 2 C atoms in total.

## Revendications

1. Tétrahydropyranne alkylé de formule (I) : 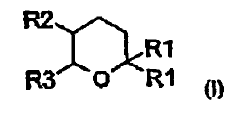 dans laquelle chaque R1 représente indépendamment l'un de l'autre soit un atome d'hydrogène, soit un radical alkyle à chaîne droite ou ramifiée ayant 1 à 3 atomes de carbone, soit un radical alcényle à chaîne droite ou ramifiée ayant 2 à 3 atomes de carbone,
R2 est un radical alkyle ou alcényle à chaîne droite ou ramifiée ayant 3 à 4 atomes de carbone, et
R3 est un radical alkyle à chaîne droite ou ramifiée ayant 1 à 5 atomes de carbone,
à l'exception du 2,6-diméthyl-3-(1-méthyléthényl)--tétrahydropyranne, du 6-méthyl-3-(1-méthyléthényl)-2-(2-méthylpropyl)-tétrahydropyranne, du 2-méthyl-3-n-propyl-tétrahydropyranne et du 2-méthyl-3-n-butyl-tétrahydropyranne.

2. Tétrahydropyranne alkylé selon la revendication 1, dans lequel chaque R1 représente indépendamment l'un de l'autre un radical méthyle, éthyle ou vinyle, R2 est un radical isopropyle, isopropényle, sec-butyle ou sec-butényle, et R3 est un radical alkyle à chaîne droite ayant 1 à 3 atomes de carbone.

3. Tétrahydropyranne alkylé selon l'une des revendications précédentes, dans lequel chaque R1 représente indépendamment l'un de l'autre un radical méthyle ou éthyle, R2 est un radical isopropyle ou sec-butyle, et R3 est un radical méthyle ou éthyle.

4. Tétrahydropyranne alkylé selon l'une des revendications précédentes, choisi dans le groupe consistant en le 2,6-diéthyl-5-isopropyl-2-méthyltétrahydropyranne, le 6-éthyl-5-isopropényl-2-méthyl-2-vinyltétrahydropyranne, le 2,6-diméthyl-5-isopropyl-2-éthyltétrahydropyranne, le 2,6-diméthyl-5-isopropényl-2-vinyltétrahydropyranne, le 2,6-diéthyl-5-sec-butyle-2-méthyltétrahydropyranne et le 2,6-diméthyl-5-sec-butyle-2-éthyltétrahydropyranne.

5. Mélange comprenant ou consistant en :
a) un, deux, trois, quatre ou plus tétrahydropyrannes alkylés selon l'une des revendications 1 à 4,
b) un ou plusieurs agents (volatils) aromatisants et/ou de sapidité, en particulier une ou plusieurs substances choisies dans le groupe consistant en les substances ayant un effet de rafraîchissement physiologique, les substances aromatiques sans effet rafraîchissant physiologique, les substances à effet trigéminal ou salivaire sans effet de rafraîchissement physiologique, et les substances modulatrices du goût,
c) et en option un support acceptable d'un point de vue cosmétique ou pharmaceutique.

6. Mélange selon la revendication 5, dans lequel
- une ou plusieurs des substances ayant un effet de rafraîchissement physiologique sont choisies dans le groupe consistant en le I-menthol, l'isopulégol, le menthoneglycérolacétal, le lactate de menthyle, les menthane-3-carboxamides substitués, les N-aryl-menthane-3-carboxamides substitués, les N-alkyl-menthane-3-carboxamides substitués, le 2-isopropyl-N-2,3-triméthylbutanamide, les cyclohexanecarboxamides substitués, le 3-menthoxy-1,2-propanediol, le carbonate de 2-hydroxyéthylmenthyle, le carbonate de 2-hydroxypropylmenthyle, l'ester menthylique de la N-acétylglycine, les esters de l'acide menthylhydroxycarboxylique, le monosuccinate de menthyle, le monoglutarate de menthyle, la 2-mercaptocyclodécanone, le 2-pyrrolidine-5-onecarboxylate de menthyle, le [N-(4-cyanométhylphényl)-p-menthanecarboxamide], le (1R,2S,5R)-2-isopropyl-5-méthyl-N-(2-(pyridine-2-yl)éthyl)cyclohexanecarboxamide, le WS-3, le WS-23, le WS-5, le WS-12, le WS-14, le TPG1^{®}, le Frescomenthe, le p-menthane-3,8-diol, le N,N-diméthylmenthylsuccinamide, la 6-isopropyl-3,9-diméthyl-1,4-dioxaspiro[4.5]décane-2-one, l'iciline et les dérivés de l'iciline, le cubébol, l'oxamate de menthyle, le N-méthyloxamate de menthyle, le N,N-diméthyloxamate de menthyle, le N-éthyloxamate de menthyle, le N,N-diéthyloxamate de menthyle, le N-propyloxamate de menthyle, le N,N-dipropyloxamate de menthyle, le N-isopropyloxamate de menthyle, le N,N-diisopropyloxamate de menthyle, le N-cyclopropyloxamate de menthyle, le N-butyloxamate de menthyle, l'ester (1R,2S,5R)-2-isopropyl-5-méthyl-cyclohexylique de l'acide morpholin-4-yl-oxo-acétique, le N-(2-méthoxyéthyl)-oxamate de menthyle, le N-(3-méthoxypropyl)-oxamate de menthyle, le N-(2-hydroxyéthyl)-oxamate de menthyle, le N-(3-hydroxypropyl)-oxamate de menthyle,
- une ou plusieurs des substances aromatiques sans effet rafraîchissant physiologique sont choisies dans le groupe consistant en les substances qui créent un goût épicé ou une sensation de chaleur ou d'échauffement sur la peau et les muqueuses, ou une sensation de chatouillement ou de picotement dans la bouche et la gorge.

7. Mélange selon l'une des revendications 5 ou 6, dans lequel une ou plusieurs substances de l'ensemble b) (i) conduisent à un effet gustatif, ou (ii) ne provoquent aucun effet gustatif.

8. Préparation servant à l'alimentation, à l'hygiène buccal ou au plaisir, ou aux préparations pharmaceutiques ou cosmétiques, comprenant (i) un ou plusieurs tétrahydropyrannes alkylés selon l'une des revendications 1 à 4, ou (ii) un mélange selon l'une des revendications 5 à 7, le constituant (i) ou, s'il est présent, le constituant (ii), se présentant en une concentration suffisante
- pour réaliser un effet de rafraîchissement physiologique sur la peau et/ou une muqueuse, et/ou
- pour conférer, modifier ou renforcer une sensation de fraîcheur dans la bouche, la gorge et/ou dans les voies respiratoires.

9. Préparation selon la revendication 8, laquelle préparation est
(i) l'une des préparations servant à l'alimentation ou au plaisir, choisie parmi les pâtisseries, les friandises, les boissons alcooliques ou non alcooliques, les boissons instantanées, les produits camés, les oeufs ou les produits dérivés des oeufs, les produits dérivés de céréales, les produits laitiers, les préparations à base de fruits, les préparations à base de légumes, les grignotines, les produits à base de graisses et d'huiles ou les émulsions de ces derniers, et pour le reste les plats cuisinés et les soupes, les épices, les mélanges d'assaisonnements, les assaisonnements à verser, les produits semi-finis, les compléments alimentaires ; ou
(ii) une préparation servant à l'hygiène buccal, de préférence à base d'un produit pour l'entretien des dents, et choisie dans le groupe consistant en les pâtes dentaires, les crèmes dentaires, les gels dentaires, les poudres dentaires, les dentifrices liquides, les mousses dentifrices, les bains de bouche, les crèmes dentaires et les bains de bouche sous forme d'un produit deux-en-un, les bonbons à sucer, les sprays buccaux, les soies dentaires et les gommes à mâcher pour les soins dentaires ; ou
(iii) une préparation pharmaceutique, la préparation étant une préparation pharmaceutique de préférence par voie orale ou nasale, de préférence sous forme de gélules, de comprimés, de dragées, de granules, de pastilles, de mélanges solides, de dispersions en phase liquide, sous forme d'émulsions, de poudres, de solutions, de pâtes ou d'autres préparations à avaler ou à mâcher ; ou
(iv) une préparation cosmétique choisie dans le groupe consistant en les savons, les détergents synthétiques, les préparations liquides pour le lavage, la douche ou le bain, les émulsions, les pommades, les pâtes, les gels, les huiles, les toners, les baumes, les sérums, les poudres, les eaux de toilette, les produits pour toilette, les eaux de Cologne, les parfums, les cires, les sticks, les produits cosmétiques à bille, les sprays (à pompe), les aérosols (moussants, non moussants ou à moussage ultérieur), les produits pour les soins des pieds, les produits pour le nettoyage et l'entretien de la barbe, les produits insectifuges, les protections solaires, les après-soleil, les produits pour le rasage, les produits après-rasage, les produits dépilatoires, les produits pour les soins des cheveux, les conditionnants, les produits pour les soins des cheveux, les toniques capillaires, les rinçages pour cheveux, les crèmes à coiffer, les pommades, les produits pour permanentes et fixateurs, les produits pour le lissage des cheveux, les mousses coiffantes, les aides au coiffage, les blondisseurs, les éclaircisseurs, les agents de conditionnement des cheveux, les mousses capillaires, les colorations pour cheveux, les produits pour les soins des ongles, les désodorisants, les antitranspirants, les bains de bouche, les douches buccales, les produits de maquillage, les démaquillants, les produits pour les soins des yeux, les cosmétiques pour les lèvres, les produits pour les soins des lèvres, les produits cosmétiques décoratifs, et les articles pour le bain et les masques.

10. Utilisation (i) d'un tétrahydropyranne alkylé de formule (I) : 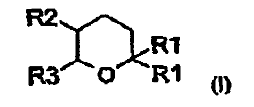 dans laquelle chaque R1 représente indépendamment l'un de l'autre un atome d'hydrogène ou un radical alkyle à chaîne droite ou ramifiée ayant 1 à 3 atomes de carbone, ou un radical alcényle à chaîne droite ou ramifiée ayant 2 à 3 atomes de carbone,
et R2 est un radical alkyle ou alcényle à chaîne droite ou ramifiée ayant 3 à 4 atomes de carbone,
et R3 est un radical alkyle à chaîne droite ou ramifiée ayant 1 à 5 atomes de carbone,
ou (ii) d'un mélange selon l'une des revendications 5 à 7
- pour produire un effet de rafraîchissement sur la peau et/ou sur une muqueuse, à des fins autres que thérapeutiques, et/ou
- pour conférer, modifier ou renforcer une sensation de fraîcheur dans la bouche, la gorge et/ou les voies respiratoires, à des fins autres que thérapeutiques.

11. Utilisation (i) d'un tétrahydropyranne alkylé de formule (I) : 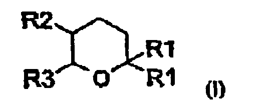 dans laquelle chaque R1 représente indépendamment l'un de l'autre soit un atome d'hydrogène, soit un radical alkyle à chaîne droite ou ramifiée ayant 1 à 3 atomes de carbone, soit un radical alcényle à chaîne droite ou ramifiée ayant 2 à 3 atomes de carbone,
et R2 est un radical alkyle ou alcényle à chaîne droite ou ramifiée ayant 3 à 4 atomes de carbone,
et R3 est un radical alkyle à chaîne droite ou ramifiée ayant 1 à 5 atomes de carbone,
ou (ii) d'un mélange selon l'une des revendications 5 à 7,
pour la fabrication d'un médicament.

12. Utilisation selon la revendication 11 pour fabriquer un médicament, le médicament à fabriquer servant à maîtriser ou atténuer des symptômes de toux, de rhume, d'inflammation, de maux de gorge ou d'enrouement.

13. Procédé non thérapeutique (a) pour produire un effet de rafraîchissement sur la peau et/ou une muqueuse et/ou (b) pour conférer, modifier ou renforcer une sensation de fraîcheur dans la bouche, la gorge et/ou les voies respiratoires, comportant l'étape suivante : application, sur la peau et/ou une muqueuse, d'une quantité suffisante pour atteindre l'effet (a) et/ou (b), (i) d'un tétrahydropyranne alkylé de formule (I) : 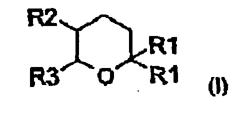 dans laquelle chaque R1 représente indépendamment l'un de l'autre soit un atome d'hydrogène, soit un radical alkyle à chaîne droite ou ramifiée ayant 1 à 3 atomes de carbone, soit un radical alcényle à chaîne droite ou ramifiée ayant 2 à 3 atomes de carbone,
et R2 est un radical alkyle ou alcényle à chaîne droite ou ramifiée ayant 3 à 4 atomes de carbone,
et R3 est un radical alkyle à chaîne droite ou ramifiée ayant 1 à 5 atomes de carbone,
ou (ii) d'un mélange selon l'une des revendications 5 à 7.

14. Procédé de préparation d'un tétrahydropyranne alkylé de formule (I) : 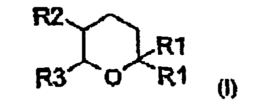 dans laquelle chaque R1 représente indépendamment l'un de l'autre soit un atome d'hydrogène, soit un radical alkyle à chaîne droite ou ramifiée ayant 1 à 3 atomes de carbone, soit un radical alcényle à chaîne droite ou ramifiée ayant 2 à 3 atomes de carbone,
et R2 est un radical alkyle ou alcényle à chaîne droite ou ramifiée ayant 3 à 4 atomes de carbone,
et R3 est un radical alkyle à chaîne droite ou ramifiée ayant 1 à 5 atomes de carbone,
comprenant les étapes suivantes :
- réaction de l'aldéhyde (B) avec le composant alcool (A) pour donner le composé tétrahydropyranne cyclisé correspondant (C),
- éventuellement, réduction du composé tétrahydropyranne (C) en le composé tétrahydropyranne (D) 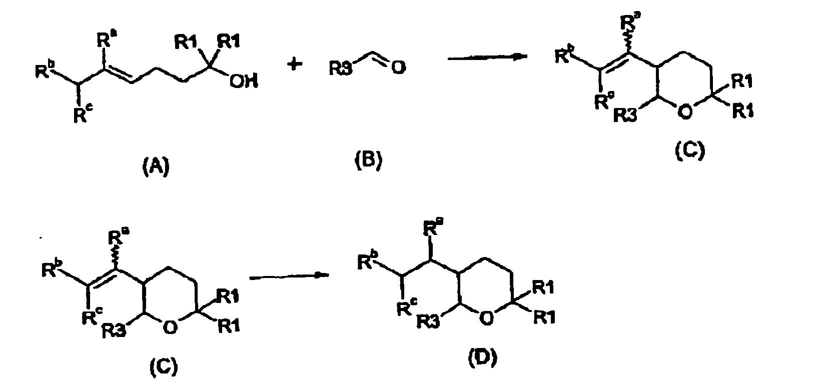
où
chacun des radicaux R1 et R3 a les significations données ci-dessus (éventuellement préférées), et
R^{a}, R^{b} et R^{c} peuvent chacun indépendamment des autres représenter un atome d`hydrogène, un radical méthyle ou éthyle, à la condition que R^{a}, R^{b} et R^{c} contiennent en tout 1 ou 2 atomes de carbone.
